# EUROPÄISCHE PATENTSCHRIFT

(11) **EP 2 049 240 B1**
(45) Veröffentlichungstag und Bekanntmachung des Hinweises auf die Patenterteilung: **13.11.2013**
(21) Anmeldenummer: 07787670.4
(22) Anmeldetag: 18.07.2007
(51) Int. Cl.: B01J 8/02, B01J 8/18

(54) **VERFAHREN ZUM LANGZEITBETRIEB EINER KONTINUIERLICH BETRIEBENEN HETEROGEN KATALYSIERTEN PARTIELLEN DEHYDRIERUNG EINES ZU DEHYDRIERENDEN KOHLENWASSERSTOFFS**
PROCESS FOR THE LONG-TERM OPERATION OF A CONTINUOUSLY OPERATED HETEROGENEOUSLY CATALYZED PARTIAL DEHYDROGENATION OF A HYDROCARBON TO BE DEHYDROGENATED
PROCÉDÉ DE FONCTIONNEMENT À LONG TERME D'UNE DÉSHYDROGÉNATION PARTIELLE À CATALYSE HÉTÉROGÈNE D'UN HYDROCARBURE À DÉSHYDROGÉNER FONCTIONNANT EN CONTINU

(30) Priorität: 28.07.2006 DE 102006035718; 28.07.2006 US 833776 P
(43) Veröffentlichungstag der Anmeldung: 22.04.2009
(73) Patentinhaber: BASF SE, 67056 Ludwigshafen (DE)
(72) Erfinder: DIETERLE, Martin, 67063 Ludwigshafen (DE); HORSTMANN, Catharina, 67056 Ludwigshafen (DE); SCHINDLER, Götz-Peter, 67071 Ludwigshafen (DE); MÜLLER-ENGEL, Klaus Joachim, 76297 Stutensee (DE); SCHEIDEL, Jens, 69493 Hirschberg (DE); ADAMI, Christoph, 69469 Weinheim (DE)
(86) Internationale Anmeldenummer: PCT/EP2007/057407
(87) Internationale Veröffentlichungsnummer: WO 2008/012249

(56) Entgegenhaltungen:
- EP-A- 0 336 622
- WO-A-01/97961
- WO-A-95/23123
- WO-A-2004/039755
- WO-A-2006/002713

## Beschreibung

Vorliegende Erfindung betrifft ein Verfahren zum Langzeitbetrieb einer kontinuierlich betriebenen heterogen katalysierten partiellen Dehydrierung eines zu dehydrierenden Kohlenwasserstoffs zu einem dehydrierten Kohlenwasserstoff, bei dem man zum Zweck der heterogen katalysierten partiellen Dehydrierung des zu dehydrierenden Kohlenwasserstoffs einen diesen in einer molaren Ausgangsmenge KW enthaltenden Reaktionsgasgemischstrom so mit erhöhter Temperatur durch ein in einer Reaktionszone RZ befindliches Katalysatorgesamtbett führt, das aus mehreren in Strömungsrichtung des Reaktionsgasgemischstroms hintereinander angeordneten Katalysatorteilbetten bestehen kann und insgesamt die Menge (Masse) M eines Dehydrierkatalysators umfasst, dass zum Betriebszeitpunkt t = to beim (einmaligen) Durchgang des Reaktionsgasgemischstroms durch das in Strömungsrichtung erste Drittel der Menge M ein Anteil von A mol-% der molaren Ausgangsmenge KW, beim Durchgang des Reaktionsgasgemischstroms durch das in Strömungsrichtung zweite Drittel der Menge M ein Anteil von B mol-% der molaren Ausgangsmenge KW und beim Durchgang des Reaktionsgasgemischstroms durch das in Strömungsrichtung letzte Drittel der Menge M ein Anteil von C mol-% der molaren Ausgangsmenge KW des zu dehydrierenden Kohlenwasserstoffs zu dehydriertem Kohlenwasserstoff mit der Maßgabe umgesetzt wird, dass A > B > C gilt und beim (einmaligen) Durchgang des Reaktionsgasgemischstroms durch das Katalysatorgesamtbett insgesamt G = (A + B + C) mol-% der in diesem enthaltenen molaren Ausgangsmenge KW an zu dehydrierendem Kohlenwasserstoff zu dehydriertem Kohlenwasserstoff dehydriert werden, wobei dem Reaktionsgasgemischstrom zwischen seinem Eintritt in den Beginn des Katalysatorgesamtbetts und seinem Austritt aus dem Ende des Katalysatorgesamtbetts gegebenenfalls Mengenströme an molekularem Sauerstoff, molekularem Wasserstoff, Wasserdampf und/oder sonstigem Inertgas als Dehydrierhilfsgase zugeführt werden, und der mit zunehmender Betriebsdauer in einem Betriebszeitpunktintervall t₀ < t < t_{R} einhergehenden Deaktivierung des Katalysatorgesamtbetts, wobei t_{R} der Betriebszeitpunkt t ist, zu dem die Dehydrierung unterbrochen und das Katalysatorgesamtbett hinter dem Betriebszeitpunkt to erstmals regeneriert wird, dadurch entgegenwirkt, dass man den Verlauf der Temperatur des Reaktionsgasgemischstroms innerhalb des Katalysatorgesamtbetts und /oder den Mengenstrom gegebenenfalls zugeführter Dehydrierhilfsgase variiert.

Der in dieser Anmeldung verwendete Begriff "dehydrierter Kohlenwasserstoff" soll Kohlenwasserstoffe umfassen, deren Moleküle wenigstens zwei ("zwei" sind anwendungstechnisch bevorzugt) Wasserstoffatome weniger enthalten, als die Moleküle eines zu dehydrierenden Kohlenwasserstoffs. Im übrigen soll der Begriff Kohlenwasserstoff Stoffe umfassen, deren Moleküle nur aus den Elementen Kohlenstoff und Wasserstoff aufgebaut ist.

Damit umfassen dehydrierte Kohlenwasserstoffe insbesondere acyclische (lineare und/oder verzweigte) und cyclische aliphatische Kohlenwasserstoffe mit einer oder mehreren C,C-Doppelbindungen im Molekül.

Beispiele für solche aliphatische dehydrierte Kohlenwasserstoffe sind Propen, iso-Buten, Ethylen, 1-Buten, 2-Buten und Butadien. D.h., zu den dehydrierten Kohlenwasserstoffen gehören insbesondere die einfach ungesättigten linearen (n-Alkene) oder verzweigten aliphatischen Kohlenwasserstoffe (z. B. iso-Alkene), sowie die Cycloalkene. Ferner sollen die dehydrierten Kohlenwasserstoffe auch die Alkapolyene (z. B. Diene und Triene) umfassen, die mehr als eine Kohlenstoff-Kohlenstoff-Doppelbindung im Molekül enthalten. Dehydrierte Kohlenwasserstoffe sollen aber auch Kohlenwasserstoffverbindungen umfassen, die ausgehend von Alkylaromaten wie Ethylbenzol oder Isopropylbenzol durch Dehydrierung des Alkylsubstituenten erhältlich sind. Das sind z. B. Verbindungen wie Styrol oder α-Methylstyrol.

Ganz generell bilden dehydrierte Kohlenwasserstoffe wertvolle Ausgangsverbindungen zur Synthese von z. B. funktionalisierten, radikalisch polymerisierbaren Verbindungen (z. B. Acrylsäure aus Propen oder Methacrylsäure aus iso-Buten) und deren Polymerisationsprodukten. Beispielsweise können solche funktionalisierten Verbindungen durch Partialoxidation von dehydrierten Kohlenwasserstoffen erzeugt werden. Dehydrierte Kohlenwasserstoffe eignen sich aber auch zur Herstellung von Verbindungen wie Methyl-tert.-butyl-ether (Folgeprodukt von iso-Buten, das z. B. als Kraftstoffadditiv zur Erhöhung der Oktanzahl geeignet ist). Dehydrierte Kohlenwasserstoffe können aber als solche auch selbst zur Polymerisation verwendet werden.

Als zu dehydrierende Kohlenwasserstoffe kommen in dieser Schrift insbesondere die acyclischen (lineare und/oder verzweigte) und cyclischen Alkane, aber auch Olefine (deren C,C-Doppelbindungszahl erhöht werden soll) in Betracht (als Beispiel sei die heterogen katalysierte partielle Dehydrierung von n-Butenen zu Butadien erwähnt).

D. h., der Begriff "zu dehydrierende Kohlenwasserstoffe" umfasst in dieser Schrift z. B. Kohlenwasserstoffe der Stöchiometrie CₙH₂ₙ₊₂ mit n > 1 bis n ≤ 20, sowie der Stöchiometrie CₙH₂ₙ mit n > 1 bis n ≤ 20, sowie der Stöchiometrie CₙH₂ₙ₋₂ mit n > 2 bis n ≤ 20, und n = ganzzahlig, insbesondere C₂- bis C₁₆-Alkane wie z. B. Ethan (zu Ethylen), Propan (zu Propylen), n-Butan, iso-Butan (zu iso-Buten), n-Pentan, iso-Pentan, n-Hexan, n-Heptan, n-Octan, n-Nonan, n-Decan, n-Undecan, n-Dodecan, n-Tridecan, n-Tetradecan, n-Pentadecan und n-Hexadecan.

Insbesondere aber gelten alle in dieser Schrift getroffenen Aussagen für C₂- bis C₆-Alkane als zu dehydrierende Kohlenwasserstoffe und ganz besonders für C₂- bis C₄-Kohlenwasserstoffe (insbesondere Alkane, und unter diesen vor allem Propan). D.h., zu dehydrierende Kohlenwasserstoffe sind in dieser Schrift vor allem Ethan, Propan, n-Butan und iso-Butan, aber auch 1-Buten und 2-Buten.

Unter einer heterogen katalysierten partiellen Dehydrierung eines Kohlenwasserstoffs soll in dieser Schrift eine (konventionelle) Dehydrierung verstanden werden, bei der wenigstens intermediär freier molekularer Wasserstoff gebildet wird und der Dehydrierschritt demzufolge endotherm verläuft (als Folgeschritt kann eine exotherme Wasserstoffverbrennung einbezogen sein). Im Unterschied dazu wird bei einer heterogen katalysierten partiellen Oxidehydrierung der dem zu dehydrierenden Kohlenwasserstoff zu entreißende Wasserstoff durch anwesenden Sauerstoff unmittelbar als Wasser (H₂O) entrissen. Der Dehydrierschritt einer heterogen katalysierten partiellen Oxidehydrierung verläuft daher grundsätzlich exotherm.

In typischer Weise benötigt eine (z. B. wie eingangs dieser Schrift beschriebene) (konventionelle) heterogen katalysierte partielle Dehydrierung eines zu dehydrierenden Kohlenwasserstoffs (z. B. von Propan) vergleichsweise hohe Reaktionstemperaturen. Typische Reaktionstemperaturen betragen 300 bis 850 °C oder bis 800 °C, bzw. 400 bis 700 °C.

Der dabei erzielte Umsatz ist normalerweise nicht kinetisch, sondern durch das thermodynamische Gleichgewicht begrenzt.

Als ausgeprägt endotherme Reaktion, bei der pro Molekül an z. B. zu Propylen dehydriertem Propan zusätzlich ein Molekül Wasserstoff erzeugt wird, begünstigen hohe Temperaturen und eine Entfernung des Reaktionsproduktes H₂ die Gleichgewichtslage im Sinne des dehydrierten Kohlenwasserstoffs als gewünschtem Zielprodukt ebenso wie eine Partialdruckerniedrigung durch inerte Verdünnung. Dabei soll in dieser Schrift als Inertgas (inertes Verdünnungsgas) generell ein Reaktionsgasgemischbestandteil verstanden werden, der sich unter den Bedingungen der entsprechenden Reaktion im wesentlichen als chemisch inert verhält und - jeder inerte Reaktionsgasgemischbestandteil für sich betrachtet - zu mehr als 95 mol-%, vorzugsweise zu mehr als 97 mol-% bzw. zu mehr als 99 mol-% chemisch unverändert erhalten bleibt. Beispiele für typische inerte Verdünnungsgase sind z. B. N₂, CO₂, H₂O, Edelgase wie He, Ne und Ar sowie Mischungen aus diesen Gasen etc..

Da bei einer heterogen katalysierten partiellen Dehydrierung eines zu dehydrierenden Kohlenwasserstoffs (z. B. Propan) der Dehydrierschritt zum dehydrierten Kohlenwasserstoff endotherm verläuft, muss die für den gewünschten Dehydrierumsatz erforderliche Reaktionswärme dem Reaktionsgas entweder vorab der heterogen katalysierten Dehydrierung und/oder im Verlauf der heterogen katalysierten Dehydrierung zugeführt werden.

In einfachster Weise lässt sich eine heterogen katalysierte partielle Dehydrierung daher wie folgt in einem adiabat betriebenen Katalysator(gesamt)bett durchführen.

Das den zu dehydrierenden Kohlenwasserstoff enthaltende Reaktionsgasgemisch wird auf seine Starttemperatur (in typischer Weise 300 bis 850 °C, häufig 400 bis 800 °C, vielfach 450 bis 750 °C, bzw. 500 bis 700 °C, oder 550 bis 650 °C) erhitzt, und anschließend in einem adiabaten Durchgang (gegen die Umgebung wärmeisoliert) durch das Katalysator(gesamt)bett geführt. Je nach Umsatz und gewählter inerter Verdünnung wird sich das Reaktionsgasgemisch beim Durchgang durch das Katalysator(gesamt)bett um etwa 30 bis 200 °C abkühlen.

Teilt man dabei das Katalysatorgesamtbett, das insgesamt die Menge M eines Dehydrierkatalysators umfasst, (gedanklich) in drei in Strömungsrichtung des Reaktionsgasgemischs aufeinander folgende Abschnitte auf, von denen jeder ein Drittel der Gesamtmenge M des Dehydrierkatalysators enthält, so wird am Anfang (d. h., bei der Inbetriebnahme des frischen bzw. des frisch regenerierten Katalysatorgesamtbetts) der eben beschriebenen heterogen katalysierten partiellen Dehydrierung beim (einmaligen) Durchgang des Reaktionsgasgemischstroms (der die molare Ausgangsmenge KW des zu dehydrierenden Kohlenwasserstoffs enthält) durch das in Strömungsrichtung erste Drittel der Menge M ein Anteil von A mol-% der molaren Ausgangsmenge KW, beim (einmaligen) Durchgang des Reaktionsgasgemischstroms durch das in Strömungsrichtung zweite Drittel der Menge M ein Anteil von B mol-% der Ausgangsmenge KW, und beim (einmaligen) Durchgang des Reaktionsgasgemischstroms durch das in Strömungsrichtung letzte Drittel der Menge M ein Anteil von C mol-% der molaren Ausgangsmenge KW des zu dehydrierenden Kohlenwasserstoffs zu dehydriertem Kohlenwasserstoff umgesetzt, wobei normalerweise in natürlicher Weise A > B > C gilt.

Insgesamt werden beim (einmaligen) Durchgang des Reaktionsgasgemischstroms durch das Katalysatorgesamtbett G = (A + B + C) mol-% der in diesem enthaltenen molaren Ausgangsmenge KW an zu dehydrierendem Kohlenwasserstoff zu dehydriertem Kohlenwasserstoff dehydriert (bezogen auf einmaligen Durchgang).

Das Katalysator(gesamt)bett kann dabei (wie ganz generell bei erfindungsgemäßen Verfahren) wenigstens ein Katalysatorfestbett, wenigstens ein Katalysatorwirbelbett, wenigstens ein Katalysatorfließbett oder eine Kombination (z. B. eine Hintereinanderanordnung) aus mehr als einem der vorgenannten Katalysatorbettvarianten sein. Erfindungsgemäß bevorzugt ist das Katalysator(gesamt)bett generell wenigstens ein Katalysatorfestbett, weshalb alle in dieser Schrift gemachten Aussagen insbesondere diesbezüglich Gültigkeit besitzen.

D. h., in einfachster Weise kann sich bei vorbeschriebener adiabater Betriebsweise das wenigstens eine Katalysatorfestbett (das Katalysatorgesamtfestbett) in einem adiabat gestalteten (gegen seine Umgebung wärmeisoliert ausgeführten) Schachtreaktor befinden. Bei einem Schachtreaktor handelt es sich um einen Reaktionsraum, der von einer den Reaktionsraum berührenden materiellen Einhüllenden umschlossen ist, die wenigstens eine erste Öffnung zur Zufuhr eines den zu dehydrierenden Kohlenwasserstoff enthaltenden Reaktionsgasgemischs in den Reaktionsraum und wenigstens eine zweite Öffnung zur Entnahme eines Produktgasstroms aus dem Reaktionsraum aufweist. Im nach außen adiabat (wärmeisoliert) gestalteten Schacht (Reaktionsraum) befindet sich vorzugsweise wenigstens ein Katalysatorfestbett, das vom Reaktionsgasgemisch durchströmt wird. Während der Verweilzeit in dem wenigstens einen Katalysatorfestbett erfolgt die angestrebte partielle Dehydrierung des zu dehydrierenden Kohlenwasserstoffs (z. B. Propan) zum dehydrierten Kohlenwasserstoff. Im Normalfall wird das wenigstens eine im adiabaten Schachtofenreaktor befindliche Katalysatorfestbett nur ein durchgehendes Katalysatorfestbett sein, und die Temperatur des den zu dehydrierenden Kohlenwasserstoff enthaltenden Reaktionsgasgemischs über die Länge dieses Katalysatorfestbettes in Strömungsrichtung sukzessive abnehmen, was den resultierenden Umsatz in natürlicher Weise beschränkt.

Mit zunehmender Starttemperatur des Reaktionsgasgemischs gehen bei vorgenannter Betriebsweise normalerweise zwar zunehmende Dehydrierumsätze G einher, doch fördern erhöhte Starttemperaturen die als Nebenreaktion unerwünschte thermische Crackung vergleichsweise stärker (die Dehydrierung (Spaltung von C-H) ist gegenüber der Crackung (Spaltung von C-C) häufig in natürlicher Weise kinetisch benachteiligt; eine Umkehr dieser Verhältnisse erfordert das Beisein von die Dehydrierung selektiv begünstigendem Katalysator und ist die Grundlage heterogen katalysierter Dehydrierungen von zu dehydrierenden Kohlenwasserstoffen).

Im vorgenannten Zusammenhang ist deshalb eine Mitverwendung von Wasserdampf als inertem Verdünnungsgas im Reaktionsgasgemisch für eine heterogen katalysierte partielle Dehydrierung vorteilhaft und in der Praxis im wesentlichen immer gegeben. Wasserdampf weist nämlich eine im Vergleich zu anderen möglichen inerten Verdünnungsgasen erhöhte molare Wärmekapazität auf, was im Rahmen der vorgenannten Mitverwendung die oben diskutierte Temperaturabnahme mindert und erhöhte Werte für G fördert.

Mit zunehmendem Wasserdampfgehalt des Reaktionsgasgemischs wächst jedoch zum einen das Volumen des Reaktionsgasgemischs (was erhöhte Investitionskosten bedingt) und zum anderen fördert ein erhöhter Wasserdampfgehalt als eine weitere unerwünschte Nebenreaktion die Dampfreformierung der Kohlenwasserstoffe, bei der sich letztere mit Wasserdampf zu CO, CO₂ und H₂ umsetzen. Die Menge an Wasserdampf, die im Einzelfall bei noch vernachlässigbarer unerwünschter Dampfreformierung mitverwendet werden kann, ist in erster Linie sowohl vom absoluten Druck (dieser wird in der Regel 0,2 bis 10 bar bzw. 0,5 bis 6 oder bis 3 bar betragen), bei dem die heterogen katalysierte Dehydrierung ausgeführt wird, als auch vom verwendeten Dehydrierkatalysator abhängig.

Grundsätzlich kann der den Dehydrierumsatz G beschränkenden Temperaturabnahme zusätzlich durch externe Temperaturlenkung entgegengewirkt werden. Unter externer Temperaturlenkung soll in dieser Schrift generell Temperaturlenkung durch indirekten Wärmeaustausch verstanden werden. Solchermaßen externe Temperaturlenkung ist in einfacher Weise im Rahmen einer heterogen katalysierten Dehydrierung eines Kohlenwasserstoffs z. B. dadurch möglich, dass man den das Katalysator (gesamt)bett enthaltenden Reaktor und damit das Katalysator(gesamt)bett selbst von außen beheizt. Beispielhaft beschrieben ist eine solche Verfahrensweise in der US-A 5,235,121.

Alternativ dazu kann man aber auch grundsätzlich am Prinzip der adiabaten Betriebsweise des Katalysatorgesamtbetts festhalten und dieses z. B. über mehrere hintereinandergeschaltete Katalysatorbetthorden oder über mehrere hintereinandergeschaltete Dehydrierreaktoren verteilen und das Reaktionsgasgemisch hinter jedem adiabat betriebenen Katalysatorteilbett einer Erwärmung durch indirekten Wärmeaustausch, d. h., durch externe Temperaturlenkung, unterwerfen.

D. h., in besonders vorteilhafter Weise lässt sich eine heterogen katalysierte Kohlenwasserstoff- (z. B. Propan)-dehydrierung in einem nach außen adiabat gestalteten Schachtreaktor dann betreiben, wenn man ihn als Hordenreaktor ausführt.

Ein solcher enthält räumlich aufeinanderfolgend z. B. mehr als ein die Dehydrierung katalysierendes Katalysator(teil)festbett im Schacht (umhüllten Reaktionsraum). Die Katalysator(teil)festbettenzahl kann z. B. 1 bis 20, zweckmäßig 2 bis 8, bzw. 3 bis 6 betragen. In der Regel sind die Katalysator(teil)festbetten dabei radial oder auch axial hintereinander angeordnet.

In besonders einfach realisierbarer Weise werden die Katalysator(teil)festbetten im nach außen adiabat gestalteten Schacht entlang dessen in Strömungsrichtung des Reaktionsgasgemischstroms weisender Achse axial hintereinander angeordnet. Sie können aber ich in den Ringspalten von im Schacht zentrisch ineinandergestellten zylindrischen Gitterrosten angeordnet sein. Auch ist es möglich, die Ringspalte im Schacht in Segmenten übereinander anzuordnen und das Reaktionsgas nach radialem Durchtritt in einem Segment in das nächste darüber oder darunter liegende Segment zu führen.

Auf seinem Weg von einem Katalysator(teil)festbett zum nächsten Katalysator(teil)festbett kann das Reaktionsgasgemisch dann zum Beispiel durch Überund/oder Durchleiten durch im ansonsten adiabat ausgestalteten Schacht zwischen den Festbetthorden im Schacht angebrachte und mittels heißen Gasen und/oder Flüssigkeiten betriebene indirekte Wärmeaustauscher (z. B. Wärmeaustauscherrippen, oder Wärmeaustauscherplatten, oder Wärmeaustauscherrohrbündel) einer indirekten Zwischenerhitzung unterworfen werden (extern gelenkter Temperaturverlauf).

In einem solchermaßen ausgestatteten Schachtofenhordenreaktor ist es für auf einmaligen Durchgang des Reaktionsgases, insbesondere bei Mitverwendung von Wasserdampf als inertem Verdünnungsgas, bezogene Dehydrierumsätze G (z. B. Propan → Propylen-Umsätze) von bis zu 50 mol-%, häufig bis zu 40 mol-%, in der Regel ausreichend (z. B. bei Verwendung der in der DE-A 10 2005 044 9216 und in der DE-A 199 37 107 beschriebenen Katalysatoren, insbesondere den beispielhaft ausgeführten), das den zu dehydrierenden Kohlenwasserstoff enthaltende Reaktionsgasgemisch auf eine Temperatur von 350 bzw. 400 bzw. 450 bis 550 °C (bevorzugt 400 bis 500 °C) vorerhitzt in den Schacht (den umhüllten, die Katalysator(teil)festbetten beherbergenden, nach außen adiabat gestalteten, Reaktionsraum) zu führen und innerhalb des Schachtes, innerhalb des Hordenreaktionsraums, durch indirekten Wärmeaustausch (extern gelenkter Temperaturverlauf) wenigstens in diesem Temperaturbereich zu halten. Der Vordruck des Reaktionsgasgemischeingangsstroms wird dabei beim Eintritt desselben in das in Strömungsrichtung erste Katalysator(teil)festbett anwendungstechnisch zweckmäßig > 1 bis 10 bar, vorteilhaft ≥ 1,5 bis 5 bar betragen. Grundsätzlich kann dieser Eintrittsdruck aber auch weniger als 1 bar betragen. Beispielsweise kann er auch 0,2 bis < 1 bar betragen.

Anwendungstechnisch noch einfacher ist es jedoch, die vorstehend geschilderte Zwischenerhitzung auf direktem Weg durchzuführen (intern gelenkter Temperaturverlauf). Dazu kann dem Reaktionsgasgemisch (dem Reaktionsgas) auf seinem Weg von Katalysator(teil)festbett zu Katalysator(teil)festbett in begrenztem Umfang zwischen zwei Katalysator(teil)festbetten jeweils ein molekularen Sauerstoff enthaltendes Gas zugesetzt werden, wodurch jeweils ein molekularen Sauerstoff, molekularen Wasserstoff und Kohlenwasserstoff sowie üblicherweise Wasserdampf enthaltender Reaktionsgasgemischstrom erzeugt wird.

Gestaltet man das in Strömungsrichtung dieses Reaktionsgasgemischstroms nachfolgende Katalysator(teil)festbett durch geeignete Auswahl der Aktivmasse dann so, dass der Dehydrierkatalysator auch die Verbrennungsreaktion von molekularem Wasserstoff mit molekularem Sauerstoff zu Wasser und/oder die Verbrennungsreaktion von im Reaktionsgasgemischstrom enthaltenem Kohlenwasserstoff zu Kohlenoxiden und Wasser katalysiert, oder setzt man dem Katalysator(teil)festbett zusätzlich zum Dehydrierkatalysator Katalysatoren zu, die diese Verbrennungen selektiv katalysieren, wird beim Durchgang des Reaktionsgasgemischs durch dieses Katalysator(teil)festbett gegebenenfalls vorab und/oder in Überlagerung zur heterogen katalysierten Dehydrierung eine begrenzte exotherme Verbrennung von im Reaktionsgasgemisch enthaltenem molekularem Wasserstoff und/oder von im Reaktionsgasgemisch enthaltendem Kohlenwasserstoff mit molekularem Sauerstoff (zu H₂O bzw. H₂O und Kohlenoxiden) erfolgen. Die dabei freigesetzte Reaktionswärme kann dann bei der nachfolgenden und/oder simultan verlaufenden endotherm ausgeformten Dehydrierung verbraucht werden. Die resultierenden Verbrennungsprodukte wie CO₂, H₂O sowie das den für die Verbrennung benötigten molekularen Sauerstoff gegebenenfalls begleitende N₂ (falls als Sauerstoffquelle z. B. Luft verwendet wird) bilden für die heterogen katalysierte Dehydrierung vorteilhaft inerte Verdünnungsgase. Die Gewichtung zwischen "Wasserstoffverbrennung" und "Kohlenwasserstoffverbrennung" kann in erster Linie durch die Katalysatorwahl beeinflusst werden. Als Katalysatoren, die vergleichsweise selektiv die Verbrennung von molekularem Wasserstoff und/oder Kohlenwasserstoff katalysieren kommen z. B. jene der Schriften US-A 4,788,371, US-A 4,886,928, US-A 5,430,209, US-A 5,530,171, US-A 5,527,979 und US-A 5,563,314 in Betracht.

Eine dominante "Wasserstoffverbrennung" ist gegenüber einer dominanten "Kohlenwasserstoffverbrennung" üblicherweise bevorzugt, da sie sowohl eine erhöhte Selektivität der Bildung der dehydrierten Kohlenwasserstoffverbindung als auch einen erhöhten Deyhdrierumsatz bezogen auf einen einmaligen Durchgang des Reaktionsgasgemischs durch den Hordenreaktor bedingt. Sie ist in der Regel dann gegeben, wenn das jeweils durchströmte Katalysator(teil)festbett nur Dehydrierkatalysator (insbesondere die in der DE-A 199 37 107 empfohlenen (insbesondere die beispielhaft ausgeführten Katalysatoren dieser DE-A) enthält, da diese in der Regel nicht nur die Dehydrierung des zu dehydrierenden Kohlenwasserstoffs (z. B. Propan) sondern auch die Verbrennung von molekularem Wasserstoff und von Kohlenwasserstoffen zu katalysieren vermögen. Die Wasserstoffverbrennung verläuft dabei in der Regel sowohl im Vergleich zur Dehydrierung des zu dehydrierenden Kohlenwasserstoffs (z. B. Propan) als auch im Vergleich zu z. B. dessen Verbrennung im Fall einer Konkurrenzsituation an diesen Katalysatoren sehr viel schneller (d. h., sie bedingen für die Verbrennung von molekularem Wasserstoff unter den gegebenen Bedingungen in der Regel die niedrigste Aktivierungsenergie).

In Abhängigkeit vom Umfang der durchgeführten Verbrennungsreaktion (d. h., auch in Abhängigkeit von der zugeführten Menge an molekularem Sauerstoff), kann der Reaktionsgesamtverlauf beim Einmaldurchgang des Reaktionsgasgemischs durch den Hordenreaktor bezüglich der integralen Wärmetönung (d. h., bezüglich der Bruttowärmetönung) sowohl endotherm (negativ), oder autotherm (im wesentlichen Null) oder exotherm (positiv) gestaltet werden.

Die Verbrennung von molekularem Wasserstoff liefert dabei etwa das Zweifache derjenigen Wärmeenergie, die zur Bildung derselben Wasserstoffmenge im Rahmen der Dehydrierung verbraucht wird.

Selbstredend kann zwischen zwei Katalysator(teil)betten auch sowohl vom Prinzip der internen als auch vom Prinzip der externen Temperaturlenkung Gebrauch gemacht werden. Ferner kann die Isothermie einer heterogen katalysierten partiellen Dehydrierung eines Kohlenwasserstoffs auch oder zusätzlich dadurch verbessert werden, dass man im Hordenreaktionsraum zwischen (und/oder in) den Katalysator(teil)festbetten geschlossene, vor ihrer Füllung günstigerweise aber nicht notwendigerweise evakuierte Einbauten (z. B. rohrförmig) anbringt. Derartige Einbauten können, wie bereits gesagt, auch ins jeweilige Katalysator(teil)festbett gestellt werden. Diese Einbauten enthalten geeignete Feststoffe oder Flüssigkeiten, die oberhalb einer bestimmten Temperatur verdampfen oder schmelzen und dabei Wärme verbrauchen und dort, wo diese Temperatur unterschritten wird, wieder kondensieren und dabei Wärme freisetzen. Grundsätzlich können außerhalb der materiellen Einhüllenden des Schachtreaktors zusätzlich fluide (gasförmige und/oder flüssige) Wärmeträger geführt werden, um die Isothermie weiter zu verbessern. Der anwendungstechnische Aufwand ist jedoch erheblich, weshalb die nach außen adiabate Ausgestaltung in der Regel bevorzugt wird.

Die beschriebene Maßnahme der internen Temperaturlenkung wird auch gerne angewendet, um das in Strömungsrichtung des Reaktionsgasgemischs dem ersten Katalysatorbett zugeführte Reaktionsgasgemisch auf die erwünschte Reaktionstemperatur zu erwärmen. Zu diesem Zweck wird bereits dem in den Schachtreaktor geführten, den zu dehydrierenden Kohlenwasserstoff enthaltenden, Reaktionsgasgemisch eine entsprechende Menge an molekularem Sauerstoff zugesetzt. Zusätzlich kann diesem Reaktionsgasgemisch vorab seines Eintritts in das erste Katalysator(fest)bett molekularer Wasserstoff zum Zweck einer solchen erwärmenden Verbrennung zugesetzt werden. Auf eine solche Wasserstoffzugabe kann aber auch verzichtet werden. Die interne Temperaturlenkung zum Zweck der Erwärmung des Reaktionsgasgemischs auf Reaktionstemperatur wird dann im wesentlichen nur durch Kohlenwasserstoffverbrennung erfolgen. Zwischen den Katalysator(teil)betten kann dem Reaktionsgasgemisch auch dadurch Wärme zugeführt werden, dass man dem Reaktionsgas überhitzten Wasserdampf zudosiert. Vielfach wird das dem ersten Katalysatorteil(fest)bett zugeführte Reaktionsgasgemisch auch auf andere Art und Weise auf Reaktionstemperatur gebracht. Beispielsweise können die Ausgangsgasströme, aus denen sich das dem ersten Katalysator(fest)bett zugeführte Reaktionsgasgemisch konstituiert, bereits entsprechende Temperaturen aufweisen. Auch können diese Ausgangsgasströme bereits molekularen Sauerstoff enthalten, so dass sich das Problem der Zudosierung eines molekularen Sauerstoff enthaltenden Gases zu einem im Schacht auf das in Strömungsrichtung erste Katalysator(fest)bett zuströmende Reaktionsgasgemisch nicht stellt oder das durch den Dehydrierreaktor zu führende und zu erwärmende Reaktionsgasgemisch wird in indirektem Wärmeaustausch zum aus dem Dehydrierreaktor geführten heißen Produktgasgemisch geführt.

Grundsätzlich sind die vorbeschriebenen Varianten einer heterogen katalysierten partiellen Dehydrierung eines zu dehydrierenden Kohlenwasserstoffs zu einem dehydrierten Kohlenwasserstoff bekannt (vgl. z. B. DE-A 10 2005 061 626, DE-A 10 2005 057 197, DE-A 10 2005 052 923, DE-A 10 2005 052 917, DE-A 10 2005 022 798, DE-A 10 2005 009 885, DE-A 10 2005 010 111, DE-A 10 2004 032129, DE-A 10 2005 013 039, WO 03/076370, DE-A 102 11 275, WO 01/96270, WO 2004/039920, DE-A 10 2004 054 657, WO 2006/050957, DE-A 10 2006 029 790, DE-A 10 2006 024 901 und den in diesen Schriften zitierten Stand der Technik).

Unabhängig davon, wie die heterogen katalysierte partielle Dehydrierung im einzelnen gestaltet und welche Art der Temperaturlenkung angewendet wird, ist allen vorstehend beschriebenen Varianten gemein, dass bei ihrer Inbetriebnahme (d. h., am frischen bzw. frisch regenerierten Katalysatorgesamtbett) für die (gedachten) Katalysator(teil)betten in der Regel nach wie vor die Dehydrierumsatzrelation A > B > C erfüllt ist, und es keiner von ihnen gelingt, die als Nebenreaktion unerwünschte thermische Crackung der Kohlenwasserstoffe vollständig zu unterdrücken. D. h., es bilden sich als unerwünschte Nebenprodukte in allen Fällen in geringen Mengen schwersiedende hochmolekulare organische Verbindungen (thermische Zersetzungsprodukte), bis hin zu elementarem Kohlenstoff, die sich auf der Katalysatoroberfläche abscheiden und selbige dadurch mit zunehmender Betriebsdauer zunehmend deaktivieren. Zwar vermag im Reaktionsgasgemisch als inertes Verdünnungsgas mitverwendeter Wasserdampf auf der Katalysatoroberfläche abgeschiedenen Kohlenstoff nach dem Prinzip der Kohlevergasung teilweise wieder zu eliminieren, aber nicht völlig zu vermeiden. In ähnlicher Weise verlängert auch im dem ersten Katalysator(teil)festbett (bzw. sonstigen Katalysator(teil)bett) zugeführten Reaktionsgasgemisch bereits mitverwendeter (zugesetzter) molekularer Wasserstoff regelmäßig die Standzeit der Dehydrierkatalysatoren, vermag deren sukzessive Deaktivierung jedoch ebenfalls nicht zu verhindern.

Eine solche Deaktivierung ist insofern von Nachteil, als mit einer zunehmenden Katalysatordeaktivierung unter ansonsten unveränderten Dehydrierbedingungen eine zunehmende Abnahme des auf den einmaligen Durchgang des Reaktionsgasgemischs durch das Katalysatorgesamtbett bezogenen Umsatzes von zu dehydrierendem Kohlenwasserstoff zu dehydriertem Kohlenwasserstoff einhergeht, was die Raum-Zeit-Ausbeute an dehydriertem Kohlenwasserstoff mindert.

Dies ist insbesondere dann von Nachteil, wenn sich an das Verfahren der heterogen katalysierten partiellen Dehydrierung des zu dehydrierenden Kohlenwasserstoffs z. B. ein Verfahren der heterogen katalysierten Partialoxidation von erzeugtem dehydriertem Kohlenwasserstoff (z. B. Propylen zu Acrolein und/oder Acrylsäure), vorzugsweise in Begleitung von nicht umgesetztem zu dehydrierendem Kohlenwasserstoff (z. B. Propan) als Inertgas in der Partialoxidation unmittelbar anschließt, mindert in einem solchen Fall eine abnehmende Raum-Zeit-Ausbeute an dehydriertem Kohlenwasserstoff doch gleichzeitig die Raum-Zeit-Ausbeute an Partialoxidationsprodukt.

Gesucht sind daher Verfahren der heterogen katalysierten partiellen Dehydrierung, bei denen der vorbeschriebenen Deaktivierung des Katalysatorgesamtbetts auf möglichst geschickte Art und Weise entgegengewirkt wird.

In der DE-A 10 2005 013 039 ist ein Verfahren der heterogen katalysierten partiellen Dehydrierung vorbeschrieben; bei dem das Katalysatorgesamtbett in Form von drei Katalysatorteilbetten auf drei hintereinandergeschaltete adiabate Reaktoren (diese drei Reaktoren bilden zusammen eine Reaktionszone RZ) verteilt ist (je Reaktor ein Katalysatorteilbett). Bevor das Reaktionsgasgemisch in das jeweilige Katalysatorteilbett eintritt, kann es auf einer Vorheizstrecke durch indirekten Wärmeaustausch auf die gewünschte Eintrittstemperatur (ins Katalysatorteilbett) erwärmt werden.

Zusätzlich wird dem Reaktionsgasgemisch vor seinem Eintritt in das jeweilige Katalysatorteilbett Luft zudosiert. Das in Strömungsrichtung in das erste Katalysatorteilbett eintretende Reaktionsgasgemisch enthält ebenfalls bereits molekularen Sauerstoff zugesetzt. Der eingesetzte Dehydrierkatalysator katalysiert gleichzeitig die Verbrennung von molekularem Wasserstoff und von Kohlenwasserstoff. Bei der Inbetriebnahme (frisches oder frisch regeneriertes Katalysatorgesamtbett) ist die Relation A > B > C erfüllt.

Um der auch in der DE-A 10 2005 013 039 beobachteten Deaktivierung des Katalysatorgesamtbetts entgegenzuwirken, enthält die DE-A 10 2005 013 039 lediglich die Empfehlung, die jeweilige Temperatureinstellung des Reaktionsgasgemischs so zu verändern, dass der auf Einmaldurchgang des Reaktionsgasgemischs durch das Katalysatorgesamtbett bezogene Dehydrierumsatz G und die daraus resultierende Raum-Zeit-Ausbeute an dehydriertem Kohlenwasserstoff im wesentlichen konstant bleiben.

Nachteilig an dieser Empfehlung ist jedoch, dass mit zunehmender Erhöhung der jeweiligen Temperatur des Reaktionsgasgemischs unter Beibehalt der Relation A > B > C zwar der Katalysatordeaktivierung entgegengewirkt werden kann, mit zunehmender Dehydriertemperatur jedoch gleichzeitig die Ursache der Deaktivierung intensiviert wird. Bei einer solchen Vorgehensweise wird deshalb bereits nach vergleichsweise kurzer Betriebsdauer der Betriebspunkt erreicht, bei dem selbst mit weiterer Erhöhung der jeweiligen Temperatur des Reaktionsgasgemischs die Raum-Zeit-Ausbeute nicht mehr aufrechterhalten werden kann.

Spätestens dann muss die Dehydrierung unterbrochen und das Katalysatorgesamtbett regeneriert werden. Derartige Regeneriermaßnahmen bestehen in der Regel darin (vgl. DE-A 100 28 582), dass man das Katalysatorgesamtbett bei erhöhter Temperatur mit einem Sauerstoff enthaltenden Gas durchströmt, um dadurch den auf der Katalysatoroberfläche abgeschiedenen Kohlenstoff quasi abzubrennen. Eine sich daran anschließende reduktive Behandlung mit molekularem Wasserstoff schließt normalerweise die Katalysatorregenerierung ab.

Problematisch in diesem Zusammenhang ist jedoch, dass die Katalysatordeaktivierung regelmäßig einen reversiblen und einen irreversiblen Anteil umfasst.

Nachteilig an der Lehre der DE-A 10 2005 013 039 ist nun ferner, dass sich bei Anwendung der dort empfohlenen Art und Weise, mit der der Katalysatordeaktivierung vorab der Katalysatorregenerierung entgegenwirkt werden soll, der vorgenannte irreversible Anteil vergleichsweise rasch bemerkbar macht. Vermutlich um diesen Sachverhalt zu vermeiden, wird in der WO 01/96008 und in der WO 95/23123 einer Katalysatordeaktivierung vorab einer Regenerierung überhaupt nicht entgegengewirkt.

Vor diesem Hintergrund bestand die Aufgabe der vorliegenden Erfindung darin, ein Verfahren zum Langzeitbetrieb einer wie eingangs beschriebenen heterogen katalysierten partiellen Dehydrierung zur Verfügung zu stellen, bei dem der Deaktivierung des Dehydrierkatalysatorgesamtbetts in einer Art und Weise entgegenwirkt wird, mit der die vorgenannten Nachteile nur noch in einem geringeren Umfang einhergehen.

Demgemäß wurde ein Verfahren zum Langzeitbetrieb einer kontinuierlich betriebenen heterogen katalysierten partiellen Dehydrierung eines zu dehydrierenden Kohlenwasserstoffs zu einem dehydrierten Kohlenwasserstoff, bei dem man zum Zweck der heterogen katalysierten partiellen Dehydrierung des zu dehydrierenden Kohlenwasserstoffs einen diesen in einer molaren Ausgangsmenge KW enthaltenden Reaktionsgasgemischstrom so mit erhöhter Temperatur durch ein in einer Reaktionszone RZ befindliches Katalysatorgesamtbett führt, das aus mehreren in Strömungsrichtung des Reaktionsgasgemischstroms hintereinander angeordneten Katalysatorteilbetten bestehen kann und insgesamt die Menge (Masse) M eines Dehydrierkatalysators umfasst, dass zum Betriebszeitpunkt t = to beim (einmaligen) Durchgang des Reaktionsgasgemischstroms durch das in Strömungsrichtung erste Drittel der Menge M ein Anteil von A mol-% der molaren Ausgangsmenge KW, beim (einmaligen) Durchgang des Reaktionsgasgemischstroms durch das in Strömungsrichtung zweite Drittel der Menge M ein Anteil von B mol-% der molaren Ausgangsmenge KW, und beim (einmaligen) Durchgang des Reaktionsgasgemischstrom durch das in Strömungsrichtung letzte Drittel der Menge M ein Anteil von C mol-% der molaren Ausgangsmenge KW des zu dehydrierenden Kohlenwasserstoffs zu dehydriertem Kohlenwasserstoff mit der Maßgabe umgesetzt wird, dass beim (einmaligen) Durchgang des Reaktionsgasgemischstroms durch das Katalysatorgesamtbett insgesamt G = (A + B + C) mol-% der in diesem enthaltenen molaren Ausgangsmenge KW an zu dehydrierendem Kohlenwasserstoff zu dehydriertem Kohlenwasserstoff dehydriert werden, wobei dem Reaktionsgasgemischstrom zwischen seinem Eintritt in den Beginn des Katalysatorgesamtbetts und seinem Austritt aus dem Ende des Katalysatorgesamtbetts gegebenenfalls Mengenströme an molekularem Wasserstoff, Wasserdampf und/oder sonstigem Inertgas als Dehydrierhilfsgase zugeführt werden, und der mit zunehmender Betriebsdauer in einem Betriebzeitpunktintervall t₀ < t < t_{R} einhergehenden Deaktivierung des Katalysatorgesamtbetts, wobei t_{R} der Betriebszeitpunkt t ist, zu dem die Dehydrierung unterbrochen und das Katalysatorgesamtbett hinter dem Betriebszeitpunkt t = t₀ erstmals regeneriert wird, dadurch entgegenwirkt, dass man den Verlauf der Temperatur des Reaktionsgasgemischstroms innerhalb des Katalysatorgesamtbetts und/oder den Mengenstrom gegebenenfalls zugeführter Dehydrierhilfsgase variiert, gefunden, das dadurch gekennzeichnet ist, dass die Variation so durchgeführt wird, dass mit zunehmendem Betriebszeitpunkt t der Anteil A abnimmt, der Anteil B ein Maximum durchläuft und der Anteil C zunimmt, und derart, dass der Gesamtumsatz G im Rahmen G ± 5 mol-% konstant bleibt..

Selbstverständlich können im großtechnischen Betrieb die Teilumsätze A, B, C beim erfindungsgemäßen Verfahren aus unterschiedlichen Gründen gewissen Schwankungen unterliegen. In diesem Fall trägt man den tatsächlichen Verlauf der Teilumsätze A, B, C über die Betriebszeit auf und legt durch die Messpunkte nach der von Legendre und Gauß entwickelten Methode der kleinsten Summe der Abweichungsquadrate jeweils eine Ausgleichskurve. Ist diese Ausgleichskurve über das Betriebszeitpunktintervall t₀ < t < t_{R} für den Teilumsatz A abnehmend sowie für den Teilumsatz C zunehmend und durchläuft sie für den Teilumsatz B ein Maximum, so ist das erfindungsgemäße Anforderungsprofil erfüllt. Unterbrechungen des erfindungsgemäßen Verfahrens finden in den Ausgleichskurven keine Berücksichtigung.

Abnehmend ist die Ausgleichskurve im relevanten Betriebszeitpunktintervall für den Teilumsatz A dann, wenn auf der Ausgleichskurve für A über das Betriebszeitpunktintervall A mit zunehmendem t entweder kontinuierlich abnimmt, oder (über das Betriebszeitpunktintervall) zwar abnimmt, zwischenzeitlich gegebenenfalls aber temporär konstant bleibt.

Zunehmend ist die Ausgleichskurve im relevanten Betriebszeitpunktintervall für den Teilumsatz C dann, wenn auf der Ausgleichskurve für C über das Betriebszeitpunktintervall C mit zunehmendem t entweder kontinuierlich zunimmt, oder (über das Betriebszeitpunktintervall) zwar zunimmt, zwischenzeitlich gegebenenfalls aber temporär konstant bleibt.

Ein Maximum (ein absolutes) durchläuft die Ausgleichskurve im relevanten Betriebszeitpunktintervall für den Teilumsatz B dann, wenn es in diesem Betriebszeitpunktintervall genau einen Betriebszeitpunkt tₘₐₓ gibt, in welchem zum einen der Wert der ersten Ableitung der Ausgleichskurve den Wert "0" hat, und zum anderen die Teilumsatzwerte B auf der Ausgleichskurve für die Betriebszeitpunkte t < tₘₐₓ kleiner als der Teilumsatz B zum Betriebszeitpunkt tₘₐₓ und die Teilumsatzwerte B auf der Ausgleichskurve für die Betriebszeitpunkte t > tₘₐₓ kleiner als und/oder gleich groß (d. h. ≤) wie der Teilumsatz B zum Betriebszeitpunkt tₘₐₓ sind.

Erfindungsgemäß zweckmäßig ist der Betriebszeitpunkt t = t₀ beim erfindungsgemäßen Verfahren der heterogen katalysierten partiellen Dehydrierung derjenige Betriebszeitpunkt eines frisch beschickten oder frisch regenerierten Dehydrierkatalysatorgesamtbetts, bei dem der Verfahrensbetrieb im wesentlichen erstmals seinen stationären Betriebszustand erreicht, der normalerweise dadurch gekennzeichnet ist, dass, bezogen auf den einmaligen Durchgang des Reaktionsgasgemischs durch das Dehydrierkatalysatorgesamtbett, G mol-% der im Reaktionsgasgemisch enthaltenen molaren Ausgangsmenge KW des zu dehydrierenden Kohlenwasserstoffs zu dehydriertem Kohlenwasserstoff umgesetzt werden. Grundsätzlich kann beim erfindungsgemäßen Verfahren der Betriebszeitpunkt t₀ aber auch hinter diesem Betriebszeitpunkt liegen.

Anwendungstechnisch zweckmäßig beträgt beim erfindungsgemäßen Verfahren zum Betriebszeitpunkt t = to, bezogen auf den Gesamtumsatz G = (A + B + C), der Teilumsatz A 45 bis 80 (bzw. bis 75) %, der Teilumsatz B 20 bis 40 % und der Teilumsatz C 0 bis bzw. 5 bis 15 %.

Erfindungsgemäß bevorzugt beträgt beim erfindungsgemäßen Verfahren zum Betriebszeitpunkt t = t₀, bezogen auf den Gesamtumsatz G = (A + B + C), der Teilumsatz A 55 bis 70 %, der Teilumsatz B 25 bis 35 % und der Teilumsatz C 7 bis 13 %.

Erfindungsgemäß besonders bevorzugt beträgt beim erfindungsgemäßen Verfahren zum Betriebszeitpunkt t = t₀, bezogen auf den Gesamtumsatz G = (A + B + C), der Teilumsatz A 55 bis 65 %, der Teilumsatz B 27 bis 33 % und der Teilumsatz C 8 bis 12 %.

Der Wert für den Gesamtumsatz G in mol-% an beim (einmaligen) Durchgang des Reaktionsgasgemischstroms durch das Katalysatorgesamtbett, bezogen auf die im Reaktionsgasgemischstrom enthaltene molare Ausgangsmenge KW an zu dehydrierendem Kohlenwasserstoff, insgesamt zu dehydriertem Kohlenwasserstoff umgesetztem zu dehydrierendem Kohlenwasserstoff, bleibt beim erfindungsgemäßen Verfahren innerhalb des Betriebszeitpunktintervalls t₀ < t < t_{R} normalerweise im Rahmen G ± 5 mol-%, vorzugsweise G ± 4 mol-%, bzw. G ± 3 mol-%, und besonders bevorzugt G ± 2 mol-%, bzw. G ± 1 mol-%, und ganz besonders bevorzugt G ± 0,75 mol-% bzw. G ± 0,50 mol-% und noch besser G ± 0,25 mol-% bzw. G ± 0,1 mol-% konstant.

Erfindungsgemäß ist es ferner günstig, wenn innerhalb des Betriebszeitpunktintervalls to < t < t_{R} mit zunehmender Betriebsdauer der Teilumsatz A unter den Teilumsatz C fällt, so dass nach einer bestimmten Betriebsdauer C > A gilt. In vielen Fällen ist es beim erfindungsgemäßen Verfahren sogar vorteilhaft, wenn sich innerhalb des relevanten Betriebszeitpunktintervalls die Abfolge der Teilumsätze A, B, C umdreht, so dass nach einer bestimmten Betriebsdauer nicht mehr A > B > C, sondern C > B > A gilt.

In der Regel unterschreitet beim erfindungsgemäßen Verfahren der Teilumsatzwert A jedoch nicht die 20 %-Marke, vorzugsweise nicht die 30 %-Marke bezogen auf den Teilumsatzwert A zum Betriebszeitpunkt t = t₀. D. h., normalerweise wird vorab eines solchen Unterschreitens das Verfahren unterbrochen und das Dehydrierkatalysatorgesamtbett regeneriert (auf diese Weise wird der irreversible Deaktivierungsanteil normalerweise minimal gehalten). Ferner überschreiten (üblicherweise aus den vorgenannten Gründen) beim erfindungsgemäßen Verfahren die Teilumsatzwerte B, C in der Regel beide nicht die 95 %-Marke, vorzugsweise nicht 85 %-Marke bezogen auf den Teilumsatzwert A zum Betriebszeitpunkt t = t₀.

In der Regel überschreiten beim erfindungsgemäßen Verfahren jedoch sowohl der Teilumsatzwert B als auch der Teilumsatzwert C die 50%-Marke bezogen auf den Teilumsatzwert A zum Betriebszeitpunkt t = to.

G wird beim erfindungsgemäßen Verfahren normalerweise 5 bis bzw. 10 bis 60 mol-%, häufig 10 bis 50 mol-%, vielfach 15 bis 40 mol-%, oder 15 bis 30 mol-%, oder 15 bis 25 mol-% betragen.

Darüber hinaus kann das erfindungsgemäße Verfahren auf alle in der Präambel dieser Schrift ausgeführten Verfahren der heterogen katalysierten partiellen Dehydrierung von Kohlenwasserstoff angewendet werden.

D. h., als Dehydrierkatalysator für das Katalysatorgesamtbett des erfindungsgemäßen Verfahrens kommen grundsätzlich alle im Stand der Technik für heterogen katalysierte Dehydrierungen bekannten Dehydrierkatalysatoren in Betracht. Sie lassen sich grob in zwei Gruppen unterteilen. Nämlich in solche, die oxidischer Natur sind (zum Beispiel Chromoxid und/oder Aluminiumoxid) und in solche, die aus wenigstens einem auf einem, in der Regel oxidischen (z. B. Zirkondioxid, Aluminiumoxid, Siliziumdioxid, Titandioxid, Magnesiumoxid, Lanthanoxid und/oder Ceroxid), Träger abgeschiedenen, in der Regel vergleichsweise edlen, Metall (z. B. einem Element aus der Platingruppe, z. B. Platin und/oder Palladium) bestehen. Unter anderem können damit alle Dehydrierkatalysatoren eingesetzt werden, die in der WO 01/96270, der EP-A 731 077, der DE-A 102 11 275, der DE-A 101 31 297, der WO 99/46039, der US-A 4,788,371, der EP-A 705 136, der WO 99/29420, der US-A 4,220,091, der US-A 5,430,220, der US-A 5,877,369, der EP-A 117 146, der DE-A 199 37 196, der DE-A 199 37 105, der US-A 3,670,044, der US-A 6,566,573 und der WO 94/29021 empfohlen werden. Weitere für das erfindungsgemäße Verfahren geeignete Dehydrierkatalysatoren enthält die WO 01/83405.

Grundsätzlich kann sowohl das Katalysatorgesamtbett als auch ein Katalysatorteilbett ausschließlich aus Dehydrierkatalysator bestehen. Selbstredend kann aber auch sowohl das Katalysatorgesamtbett als auch ein Katalysatorteilbett den Dehydrierkatalysator in mit inertem Material verdünnter Form aufweisen.

Da das Katalysatorgesamtbett für das erfindungsgemäße Verfahren bevorzugt ein Festbett (Alternativen sind z. B. das Wirbelbett und das Fließbett) ist (alle in dieser Schrift gemachten Aussagen gelten insbesondere für die Festbettvariante), soll unter einem Dehydrierkatalysator in dieser Schrift im besonderen ein Formkörper verstanden werden, dessen Längstausdehnung L (längste direkte Verbindungslinie zweier auf der Oberfläche des Formkörpers befindlicher Punkte) 0,1 bzw. 1 bis 30 mm, vorzugsweise 1 bis 20 mm und besonders bevorzugt 1 bis 10 mm bzw. 1 bis 5 mm beträgt und der im nachfolgend beschriebenen Versuch, bezogen auf einmaligen Durchgang des Reaktionsgasgemischs durch das Reaktionsrohr, wenigstens 5 mol-% des im Reaktionsgas enthaltenen Propan zu Propylen dehydriert:

Ein Reaktionsrohr aus Stahl der EN Werkstoffnummer 1.4835 mit einer Wanddicke von 2 mm und einem Innendurchmesser von 35 mm sowie einer Länge von 80 cm wird wie folgt befüllt:
50 ml aus einer Schüttung des entsprechenden Dehydrierkatalysators werden im Reaktionsrohr mittig platziert. Oberhalb und unterhalb der Schüttung aus Katalysatorformkörper wird das Reaktionsrohr jeweils mit einer Schüttung aus Steatitkugeln (Inertkugeln) mit einem Kugeldurchmesser von 1,5 mm bis 2,5 mm aufgefüllt. Ein Gitterrost trägt die gesamte Schüttung. Von außen wird das Reaktionsrohr auf seiner gesamten Länge auf einer Temperatur von 550 °C gehalten. Das Reaktionsrohr wird mit einem Gemisch aus Propan und Wasserdampf im Volumenverhältnis 2 (Propan) zu 1 (Wasserdampf) mit einer Propanbelastung der Schüttung aus Katalysatorformkörpern von 1000 Nl/l•h beschickt. Der in das Reaktionsrohr geführte Reaktionsgasgemischstrom ist auf eine Temperatur von 550 °C vorerwärmt. Besonders bevorzugt sind für das erfindungsgemäße Verfahren Dehydrierkatalysatoren, bei denen unter den vorstehenden Rahmenbedingungen die Summenselektivität der Bildung der Nebenprodukte Ethan, Ethylen und Methan ≤ 5 mol-%, bezogen auf umgesetztes Propan, beträgt.

Unter der Belastung eines einen Reaktionsschritt katalysierenden Katalysatorbetts mit Reaktionsgas soll in dieser Schrift ganz generell die Menge an Reaktionsgas in Normlitern (= Nl; das Volumen in Litern, das die entsprechende Reaktionsgasmenge bei Normalbedingungen (0 °C, 1 atm) einnehmen würde) verstanden werden, die pro Stunde durch einen Liter Katalysatorbett geführt wird. Die Belastung kann aber auch nur auf einen Bestandteil des Reaktionsgases bezogen sein. Dann ist es die Menge dieses Bestandteils in Nl/l•h, die pro Stunde durch einen Liter des Katalysatorbetts geführt wird (reine Inertmaterialschüttungen werden nicht zu einem Katalysatorfestbett gerechnet). Die Belastung kann auch nur auf die in einem Katalysatorbett, das den eigentlichen Katalysator mit Inertmaterial verdünnt enthält, enthaltene Menge an Katalysator bezogen sein (dies wird dann explizit vermerkt).

Grundsätzlich kann das erfindungsgemäße Verfahren bei Belastungen des Katalysatorgesamtbetts (bezogen auf die darin enthaltene Gesamtmenge M an Dehydrierkatalysator) sowohl mit Reaktionsgas als auch mit dem darin enthaltenen zu dehydrierenden Kohlenwasserstoff (z. B. Propan) von 100 bis 10 000 h⁻¹ (Nl/l•h, verkürzt: h⁻¹), häufig von 300 bis 5000 h⁻¹, das heißt, vielfach 500 bis 3000 h⁻¹ (im wesentlichen unabhängig vom gewünschten Umsatz an zu dehydrierendem Kohlenwasserstoff) betrieben werden.

Zur Verdünnung des Dehydrierkatalysators im Katalysatorgesamtbett und/oder Katalysatorteilbett kommen als inerte Materialien z. B. gebrannte Tone (Aluminiumsilicate) oder Steatit (z. B. Typ C 220 der Fa. CeramTec), oder sonstige (vorzugsweise im wesentlichen an Poren freie) Hochtemperaturchemikalien wie Aluminiumoxide, Siliciumdioxid, Titandioxid, Magnesiumoxid, Lanthanoxid, Ceroxid, Zinkaluminiummischoxid, Thoriumdioxid, Zirkondioxid, Siliciumcarbid oder sonstige Silicate wie Aluminium und/oder Magnesiumsilicat und Mischungen aus vorgenannten Materialien in Betracht. Formkörper aus vorgenannten Materialien kommen beim erfindungsgemäßen Verfahren aber nicht nur zur Verdünnung des Katalysatorgesamtfestbetts und/oder von Katalysatorteilfestbetten sondern auch als inerte Deck- und gegebenenfalls Abschlussschüttung des Katalysatorgesamtfestbetts und/oder von Katalysatorteilfestbetten in Betracht.

Mit Vorteil sollte eine solche inerte Deck- und/oder Abschlussschüttung aus inerten Formkörpern beim erfindungsgemäßen Verfahren so beschaffen sein, dass im Reaktionsgasgemischstrom beim Durchströmen dieser Inertschüttung ≤ 3 mol-%, besser ≤ 2 mol-%, noch besser ≤ 1 mol-% oder 0 mol-% des darin enthaltenen zu dehydrierenden Kohlenwasserstoff zu dehydriertem Kohlenwasserstoff umgesetzt werden.

Im besonderen können für das erfindungsgemäße Verfahren als Dehydrierkatalysator für das Katalysatorgesamtbett die Katalysatoren gemäß Beispiel 1, Beispiel 2, Beispiel 3 und Beispiel 4 der DE-A 199 37 107 eingesetzt werden.

Dabei handelt es sich um Dehydrierkatalysatoren, die 10 bis 99,9 Gew.-% Zirkondioxid, 0 bis 60 Gew.-% Aluminiumoxid, Siliciumdioxid und/oder Titandioxid und 0,1 bis 10 Gew.-% mindestens eines Elements der ersten oder zweiten Hauptgruppe, eines Elements der dritten Nebengruppe, eines Elements der achten Nebengruppe des Periodensystems der Elemente, Lanthan und/oder Zinn enthalten, mit der Maßgabe, dass die Summe der Gewichtsprozente 100 Gew.-% ergibt.

In einer bevorzugten Ausführungsform enthalten vorgenannte Dehydrierkatalysatoren wenigstens ein Element der VIII. Nebengruppe, mindestens ein Element der I. und II. Hauptgruppe, mindestens ein Element der III. und/oder IV. Hauptgruppe und mindestens ein Element der III. Nebengruppe einschließlich Lanthaniden und Actiniden. Als Element der VIII. Nebengruppe enthält die Aktivmasse der Dehydrierkatalysatoren bevorzugt Platin und/oder Palladium, besonders bevorzugt Platin. Als Elemente der I. und II. Hauptgruppe enthält die Aktivmasse der vorgenannten Dehydrierkatalysatoren bevorzugt Kalium und/oder Cäsium. Als Elemente der III. Nebengruppe einschließlich der Lanthaniden und Actiniden enthält die Aktivmasse der vorgenannten Dehydrierkatalysatoren bevorzugt Lanthan und/oder Cr. Als Elemente der III. und/oder IV. Hauptgruppe enthält die Aktivmasse der vorgenannten Dehydrierkatalysatoren bevorzugt ein oder mehrere Elemente aus der Gruppe bestehend aus Bor, Gallium, Silizium, Germanium, Indium, Zinn und Blei, besonders bevorzugt Zinn. Ganz besonders bevorzugt enthält die Aktivmasse der vorgenannten Dehydrierkatalysatoren jeweils wenigstens einen Vertreter der vorgenannten Elementgruppen.

Generell kann es sich beim Dehydrierkatalysator für ein Katalysatorgesamtfestbett und/oder Katalysatorteilfestbett um Katalysatorstränge (Durchmesser typisch 0,1 bzw. 1 bis 10 mm, bevorzugt 1,5 bis 5 mm; Länge typisch 1 bis 20 mm, bevorzugt 3 bis 10 mm), Tabletten (vorzugsweise gleiche Abmessungen wie bei den Strängen) und/oder Katalysatorringe (Außendurchmesser und Länge jeweils typisch 2 bis 30 mm oder bis 10 mm, Wandstärke zweckmäßig 1 bis 10 mm, oder bis 5 mm, oder bis 3 mm) handeln.

Grundsätzlich bestehen zur Verwendung im Festbett weder hinsichtlich der Katalysatorgeometrie (insbesondere im Fall von Trägerkatalysatoren) noch hinsichtlich der Geometrie der inerten Formkörper Einschränkungen. Besonders häufige Geometrien sind Vollzylinder, Hohlzylinder (Ringe), Kugeln, Kegel, Pyramiden und Würfel sowie Stränge, Wagenräder, Sterne und Monolithe.

Die Längstausdehnung der Katalysatorformkörper sowie der Inertformkörper (längste direkte Verbindungslinie zweier auf der Formkörperoberfläche befindlicher Punkte) kann dabei 0,5 mm bis 100 mm, oft 1,5 mm bis 80 mm, und vielfach 3 mm bis 50 mm bzw. bis 20 mm betragen).

Schließlich seien auch noch die Dehydrierkatalysatoren der deutschen Anmeldung Nr. 10 2005 044 916 für ein erfindungsgemäßes Katalysatorgesamtfestbett und/oder Katalysatorteilfestbett als besonders geeignet empfohlen.

Die Regenerierung der für das erfindungsgemäße Verfahren empfohlenen Dehydrierkatalysatoren kann insbesondere so erfolgen, dass man bei einer Eintrittstemperatur von 300 bis 600 °C (in Extremfällen gegebenenfalls auch bis 750 °C), häufig 400 bis 450 °C, zunächst in ersten Regenerierstufen mit molekularem Stickstoff und/oder Wasserdampf (bevorzugt) verdünnte Luft durch das Katalysatorgesamt(fest)bett leitet. Die Katalysatorbelastung mit Regeneriergas kann (bezogen auf die Dehydrierkatalysatorgesamtmenge M) dabei z. B. 50 bis 10000 h⁻¹ und der Sauerstoffgehalt des Regeneriergases 0,1 oder 0,5 bis 20 Vol.-% betragen. In darauffolgenden weiteren Regenerierungsstufen kann unter ansonsten gleichen Regenerierungsbedingungen als Regeneriergas Luft verwendet werden. Anwendungstechnisch zweckmäßig empfiehlt es sich, das den Dehydrierkatalysator enthaltende Katalysatorgesamt(fest)bett vor Beginn der Regenerierung mit Inertgas (z. B. N₂, z. B. Industriestickstoff mit bis zu 1 Vol.-% O₂, H₂O oder deren Gemische) zu spülen. Als Abschluss der Regenerierung ist es in der Regel empfehlenswert, das Katalysatorgesamtbett noch mit reinem molekularem Wasserstoff (Reinheit > 99 Vol.-%) oder mit durch Inertgas (vorzugsweise Wasserdampf und/oder Stickstoff) verdünntem molekularem Wasserstoff (der Wasserstoffgehalt sollte ≥ 1 Vol.-% betragen) im ansonsten gleichen Bedingungsraster zu regenerieren.

Grundsätzlich kann die Reaktionszone RZ bei erfindungsgemäßen Verfahren nur einen oder mehrere Dehydrierreaktoren umfassen. Diese können hintereinander und/oder parallel geschaltet betrieben werden. In entsprechender Weise kann sich das Katalysatorgesamtbett in nur einem Dehydrierreaktor oder in Form von Katalysatorteilbetten auf mehrere Reaktoren verteilt befinden. Befindet sich das Katalysatorgesamtbett in nur einem Dehydrierreaktor, kann es sich grundsätzlich um nur ein in Strömungsrichtung ohne Unterbrechung durchgehendes Katalysatorbett handeln.

Das Katalysatorgesamtbett kann in diesem Fall in Strömungsrichtung aber auch z. B. durch reine Inertmaterial-Abschluss- und/oder -Vorbetten (z. B. Formkörperschüttungen) bedingte Unterbrechungen der Aktivmasse aufweisen. Das Katalysatorgesamtbett kann aber auch z. B. aus in einem Reaktor radial oder axial hintereinander angeordneten Teilfestbetten bestehen, zwischen denen sich an Festkörper freier Zwischenraum befindet. Dies ist z. B. dann der Fall, wenn es sich bei der Reaktionszone RZ um einen Hordenreaktor handelt. Selbstverständlich kann eine Reaktionszone aber auch mehrere Hordenreaktoren umfassen. Sie kann aber auch aus einer Reaktorverschaltung bestehen, die einen oder mehrere Dehydrierreaktoren umfasst, die nur ein Dehydrierkatalysatorteilbett (z. B. ein Dehydrierkatalysatorteilfestbett) enthalten, und einen oder mehrere Dehydrierreaktoren, die mehr als ein Dehydrierkatalysatorteilbett enthalten (z. B. in Form von Katalysatorteilfestbetthorden).

Um der mit zunehmender Betriebsdauer in einem Betriebszeitpunktintervall tₒ < t < t_{R} einhergehenden Deaktivierung des Katalysatorgesamtbetts erfindungsgemäß entgegenzuwirken, kommen in erster Linie alle in der Präambel dieser Schrift bereits beschriebenen Methoden der externen und internen Lenkung der Temperatur des Reaktionsgasgemischs auf seinem Weg durch das Katalysatorgesamtbett in Betracht, einschließlich der Einstellung der Temperatur des in das Katalysatorgesamtbett eintretenden, die molare Ausgangsmenge KW des zu dehydrierenden Kohlenwasserstoffs enthaltenden Reaktionsgasgemischstroms. Grundsätzlich kann aber auch z. B. Partialdruckänderung durch z. B. Variation der Inertgasverdünnung angewendet werden. Häufig werden die verschiedenen möglichen Steuerungsmethoden aber auch gemeinsam angewendet. Dazu zählt auch die Entfernung des Reaktionsproduktes H₂. In einfacher Weise kann man das erfindungsgemäße Ziel z. B. dadurch erreichen, dass man beim erfindungsgemäßen Verfahren das Katalysatorgesamtbett sowohl im Umfang des in Strömungsrichtung des Reaktionsgasgemischstroms ersten Drittels der im Katalysatorgesamtbett insgesamt enthaltenen Menge M an Dehydrierkatalysator, als auch im Umfang des in Strömungsrichtung zweiten und letzten Drittels dieser Menge M adiabat gestaltet, jedoch mit der Möglichkeit, den Wärmeinhalt des Reaktionsgasgemischs vor dem Eintritt in das jeweilige adiabat gestaltete Drittel zu verändern.

Anwendungstechnisch zweckmäßig wird man zum Betriebszeitpunkt t₀ dann so verfahren, dass die Temperatur T₁ des Reaktionsgasgemischs beim Eintritt in das in Strömungsrichtung erste Drittel der Menge M größer ist, als die Temperatur T₂ des Reaktionsgasgemischs beim Eintritt des selben in das zweite Drittel der Menge M, und T₂ größer ist, als die Temperatur T₃ des Reaktionsgasgemischs beim Eintritt des selben in das in Strömungsrichtung letzte Drittel der Menge M.

Der zunehmenden Deaktivierung des Katalysatorgesamtbetts im Betriebszeitpunktintervall tₒ < t < t_{R} lässt sich nun in erfindungsgemäß einfacher Weise dadurch entgegenwirken, dass man sowohl T₁, als auch T₂ und T₃ in im Sinne der erfindungsgemäßen Zielsetzung aufeinander abgestimmter Weise erhöht. Die Temperaturzuwachsrate von T₃ liegt dabei normalerweise stetig oberhalb der Temperaturzuwachsrate von T₁. Die Temperaturzuwachsrate von T₂ liegt dabei dagegen normalerweise anfänglich oberhalb der Temperaturzuwachsrate von T₃, um im weiteren Betriebsverlauf unter die Temperaturzuwachsrate von T₃ zu fallen und sich der geringeren Zuwachsrate von T₁ immer mehr anzunähern.

Auf die vorbeschriebene Art und Weise lässt sich nicht nur der angestrebte beim (einmaligen) Durchgang des den zu dehydrierenden Kohlenwasserstoff in einer molaren Ausgangsmenge KW enthaltenden Reaktionsgasgemischstroms durch das Katalysatorgesamtbett zu dehydriertem Kohlenwasserstoff umgesetzte molare Anteil G (ausgedrückt in mol-% von KW) von KW möglichst lange im wesentlichen unverändert aufrechterhalten, sondern die aus dieser Betriebsweise resultierende Deaktivierung ist über eine vergleichsweise lang anhaltende Betriebsdauer dominant reversible, d. h., durch anschließende Anwendung der bereits beschriebenen Regenerierverfahren weitestgehend wieder aufhebbar.

Erfindungsgemäß wesentlich ist, dass der reale Verdünnungsgrad der verschiedenen Drittelteilmengen der Gesamtmenge M an Dehydrierkatalysator mit Inertmaterial im realen Katalysatorgesamtbett bei der erfindungsgemäßen Verfahrensweise (insbesondere bei der ebenda beschriebenen Verfahrensvariante) sowohl identisch als auch unterschiedlich sein kann. Grundsätzlich können alle Drittelteilmengen von M auch unverdünnt im Katalysatorgesamtbett enthalten sein. Selbstredend kann sich der Verdünnungsgrad des Dehydrierkatalysators auch innerhalb einer Drittelteilmenge von M in Strömungsrichtung ändern.

In gewisser Weise ist das erfindungsgemäße Verfahren so angelegt, dass der in Strömungsrichtung durch eine beliebig kleine Teilmenge von M bedingte Beitrag zu G normiert auf diese (d. h., geteilt durch diese) Teilmenge mit der Maßgabe einen Maximalwert durchläuft, dass der Ort dieses normierten Maximalbeitrags zu G über den erfindungsgemäßen Langzeitbetrieb im Betriebszeitpunktintervall to < t < t_{R} mit zunehmendem Betriebszeitpunkt t das Dehydrierkatalysatorgesamtbett in Strömungsrichtung vom Eingang in das Katalysatorgesamtbett ausgehend zum Ausgang des Reaktionsgasgemischstroms (des Produktgasgemischstroms) aus dem Katalysatorgesamtbett hin durchläuft.

In besonders einfacher Weise ist die ebenda beschriebene erfindungsgemäße Langzeitbetriebsweise in einem adiabat gestalteten Hordenreaktor realisierbar. Dabei kann das jeweilige Katalysatorteilbett im Reaktionsraum sowohl ein Wirbelbett, oder ein Fließbett, oder ein Festbett sein. Selbstredend können Wirbelbett und z. B. Festbett, oder Fließbett und Festbett oder jedwede andere Kombination der genannten Katalysatorbetttypen im Reaktionsraum auch kombiniert enthalten sein. In anwendungstechnisch zweckmäßiger Weise wird der Reaktionsgasgemischstrom dann auf seinem Weg von einer Katalysatorteilbetthorde zur nächsten Katalysatorteilbetthorde z. B. durch Überleiten über mit heißen Gasen bzw. Wasserdampf erhitzte Wärmeaustauscherrippen oder durch leiten durch mit heißen Brenngasen bzw. Wasserdampf erhitzte Rohre bzw. mit heißen Gasen erhitzte Wärmeaustauscherplatten im ansonsten adiabat gestalteten Hordenreaktionsraum der für diese erfindungsgemäße Verfahrensvariante erforderlichen Zwischenerhitzung unterworfen. In entsprechender Weise kann auch die Temperatur des dem Eingang in das Katalysatorgesamtbett zugeführten, die Ausgangsmenge KW enthaltenden, Reaktionsgasgemischstroms variiert werden. In besonders einfacher Weise wird der Hordenreaktor drei identische Katalysatorhorden enthalten. Prinzipiell kann der Hordenreaktor aber auch sechs, oder neun, oder zwölf oder eine andere Anzahl von identisch oder unterschiedlich beschickten Katalysatorhorden enthalten. Eine Zwischenerhitzung kann dabei zwischen allen aufeinanderfolgenden Horden oder nur zwischen einer Teilmenge von aufeinanderfolgenden Horden vorgenommen werden.

Anwendungstechnisch besonders zweckmäßig handelt es sich bei vorgenanntem Hordenreaktor um einen reinen Katalysatorfestbetthordenreaktor. Im einfachsten Fall sind die Katalysatorteilfestbetten im Reaktionsraum dabei in einer der Anzahl der Teilfestbetten entsprechenden Anzahl von Horden in Strömungsrichtung des Reaktionsgasgemischstroms axial hintereinander oder in den Ringspalten von zentrisch ineinandergestellten zylindrischen Gitterrosten räumlich nacheinander angeordnet. Es ist jedoch auch möglich, die Ringspalte im Reaktionsraum in Segmenten übereinander anzuordnen und den Reaktionsgasgemischstrom nach radialem Durchtritt in einem Segment in das nächste darüber- oder darunter liegende Segment zu führen. In einfachster Form sind die Katalysatorteil(fest)betthorden in einem adiabaten Schachtofenreaktor untergebracht.

Erfindungsgemäß bevorzugt befindet sich sowohl vor als auch hinter einem Katalysatorteilfestbett in einem für das erfindungsgemäße Verfahren eingesetzten Festbetthordenreaktor eine Schüttung aus inerten Formkörpern. Dies rührt zum einen daher, dass die Dehydrierkatalysatorformkörper häufig eine geringere Ausdehnung als die Maschenweite des zugehörigen Gitter(trage)rostes aufweisen. Mittels einer Nachschüttung aus inerten Formkörpern geeigneter Größe kann dem entgegengewirkt werden.

Eine in Strömungsrichtung vorgelagerte Schüttung aus inerten Formkörpern erweist sich unter anderem unter dem Gesichtspunkt eines möglichst homogenen (sowohl in Bezug auf Temperatur als auch Reaktionsgasgemischzusammensetzung) Reaktionsgasgemischstroms als vorteilhaft. Dies insbesondere dann, wenn der dem Katalysatorteilfestebett zugeführte Reaktionsgasgemischstrom durch Zusammenführung verschiedener Einzelgasströme erzeugt worden ist.

Letzteres ist zum Beispiel dann von Bedeutung, wenn die für vorstehend beschriebene Ausführungsvariante des erfindungsgemäßen Verfahrens zwischen den Dehydrierkatalysatorteilbetten anzuwendende indirekte Temperaturlenkung wenigstens teilweise durch eine direkte Temperaturlenkung ersetzt wird. Anwendungstechnisch bevorzugt wird eine zwischen zwei Katalysatorteilbetten anzuwendende Variation der Temperaturlenkung beim erfindungsgemäßen Verfahren ausschließlich über den Weg einer direkten Temperaturlenkung verwirklicht.

Dazu setzt man dem Reaktionsgasgemischstrom auf seinem erfindungsgemäßen Weg durch die Reaktionszone RZ z. B. nach Durchströmung des ersten Katalysatorteilbettes und gegebenenfalls zwischen den nachfolgenden Katalysatorteilbetten in über die Betriebsdauer des erfindungsgemäßen Verfahrens variierendem begrenztem Umfang ein molekularen Sauerstoff enthaltendes Gas zu.

Dieses kann z. B. reiner molekularer Sauerstoff oder ein Gemisch aus Inertgas und molekularem Sauerstoff (z. B. Luft) sein. Je nach verwendetem Dehydrierkatalysator wird so, wie in der Präambel zu dieser Schrift bereits ausgeführt, eine in entsprechender Weise variierende begrenzte Verbrennung der im Reaktionsgasgemisch enthaltenen Kohlenwasserstoffe, gegebenenfalls bereits auf der Katalysatoroberfläche abgeschiedener Kohle beziehungsweise kohleähnlicher Verbindungen und/oder von im Verlauf der konventionellen heterogen katalysierten Dehydrierung (z. B. einer Propandehydrierung) gebildetem und/oder dem Reaktionsgas zugesetztem Wasserstoff bewirkt (es kann auch anwendungstechnisch zweckmäßig sein, im Hordenreaktionsraum (in der Reaktionszone RZ) Katalysatorbetten einzufügen, die mit Katalysator beschickt sind, der spezifisch (selektiv) die Verbrennung von Wasserstoff (und/oder von Kohlenwasserstoff) katalysiert (als solche Katalysatoren kommen zum Beispiel jene der Schriften US-A 4,788,371, US-A 4,886,928, US-A 5,430,209, US-A 5,530,171, US-A 5,527,979 und US-A 5,563,314 in Betracht; beispielsweise können solche Katalysatorbetten in alternierender Weise zu den Dehydrierkatalysator enthaltenden Betten im Hordenreaktionsraum untergebracht sein)). Die in Entsprechung zur Variation der zudosierten Menge an molekularem Sauerstoff einhergehende Variation der durch die begrenzte Verbrennung freigesetzten Reaktionswärme bedingt dann die erfindungsgemäß geforderte Variation der Dehydrierumsatzanteile. Im Unterschied dazu wird man anwendungstechnisch zweckmäßig den Sauerstoffgehalt des die Ausgangsmenge KW an zu dehydrierendem Kohlenwasserstoff enthaltenden Reaktionsgasgemischstroms im Rahmen eines erfindungsgemäßen Langzeitbetriebs üblicherweise im wesentlichen konstant halten und lediglich die Temperatur dieses (Eingangs)Reaktionsgasgemischstroms variieren (in der Regel durch indirekten Wärmeaustausch). Die Maßnahme der direkten Temperaturlenkung kann jedoch nicht nur im Fall eines Hordenreaktors als Reaktionszone RZ angewendet werden. Sie kann vielmehr auch im Fall eines einzigen in Strömungsrichtung des Reaktionsgasgemischstroms ununterbrochenen Dehydrierkatalysatorbetts in der Reaktionszone RZ angewendet werden. Dies ist in anwendungstechnisch einfacher Weise z. B. dadurch möglich, dass man in das z. B. Katalysatorfestbett auf unterschiedlichen Katalysatorbettlängen Sauerstoffzudosierungssonden (-lanzen) eingeführt hat, über die dem Reaktionsgasgemischstrom ein molekularen Sauerstoff enthaltendes Gas zugeführt werden kann.

In einfacher Weise kann man das erfindungsgemäße Ziel deshalb auch dadurch erreichen, dass man beim erfindungsgemäßen Verfahren das Katalysatorgesamtbett sowohl im Umfang des in Strömungsrichtung des Reaktionsgasgemischstroms ersten Drittels der im Katalysatorgesamtbett insgesamt enthaltenen Menge M an Dehydrierkatalysator, als auch im Umfang des in Strömungsrichtung zweiten und letzten Drittels dieser Menge M adiabat gestaltet, jedoch mit der Möglichkeit, den Wärmeinhalt des Reaktionsgasgemischs vor dem Eintritt in das in Strömungsrichtung erste adiabat gestaltete Drittel durch indirekte Temperaturlenkung und beim Eintritt in das in Strömungsrichtung zweite bzw. dritte adiabat gestaltete Drittel durch direkte Temperaturlenkung (z. B. durch Variation des Mengenstroms an vor dem Eintritt des Reaktionsgasgemischstroms in das zweite bzw. dritte adiabat gestaltete Drittel diesem jeweils zugeführtem molekularen Sauerstoff enthaltenden Gas) zu verändern.

Anwendungstechnisch zweckmäßig wird man zum Betriebszeitpunkt t₀ dann so verfahren, dass die höchste Temperatur T₁* des Reaktionsgasgemischstroms beim Durchströmen des in Strömungsrichtung ersten Drittels der Menge M größer ist, als die höchste Temperatur T₂* des Reaktionsgasgemischstroms beim Durchströmen des in Strömungsrichtung zweiten Drittels der Menge M, und T₂* größer ist, als die maximale Temperatur T₃* des Reaktionsgasgemischstroms beim Durchströmen des in Strömungsrichtung letzten Drittels der Menge M.

Die Einstellung von T₁* ist in einfacher Weise durch Einstellung des Wärme- und molekularen Sauerstoffinhalts des Reaktionsgasgemischstroms beim Eintreten desselben in das in Strömungsrichtung erste Drittel der Menge M möglich. Die Relativlage von T₂* und T₃* sowohl zueinander als auch bezüglich T₁* wird dann durch die dem Reaktionsgasgemischstrom zwischen dem in Strömungsrichtung ersten und zweiten Drittel der Menge M bzw. zwischen dem in Strömungsrichtung zweiten und letzten (dritten) Drittel der Menge M zudosierten Mengenströme an molekularem Sauerstoff enthaltendem Gas festgelegt. In einfacher Weise wird man in beiden Fällen das selbe molekularen Sauerstoff enthaltende Gas (z. B. reiner molekularer Sauerstoff (Reinheit ≥ 99 mol-%), oder Luft oder ein sonstiges Gemisch aus Inertgas und molekularem Sauerstoff) verwenden. Die angestrebte Relativlage von T₂*, T₃* zueinander wird zum Betriebszeitpunkt t = t₀ üblicherweise dann besonders einfach erreicht, wenn man zu diesem Betriebszeitpunkt die zudosierten Mengenströme an molekularem Sauerstoff identisch wählt.

Der zunehmenden Deaktivierung des Katalysatorgesamtbetts im Betriebszeitpunktintervall t₀ < t < t_{R} lässt sich nun in erfindungsgemäß einfacher Weise dadurch entgegenwirken, dass man sowohl den Wärmeinhalt (die Temperatur) des Reaktionsgasgemischstroms beim Eintritt desselben in das in Strömungsrichtung erste Drittel der Menge M als auch die zwischen dem in Strömungsrichtung ersten und zweiten Drittel der Menge M (Mengenstrom 1) bzw. zwischen dem in Strömungsrichtung zweiten und letzten (dritten) Drittel der Menge M (Mengenstrom 2) zudosierten Mengenströme an molekularem Sauerstoff (bzw. an diesen enthaltendem Gas) in im Sinne der erfindungsgemäßen Zielsetzung aufeinander abgestimmter Weise erhöht.

Die Zuwachsraten der Mengenströme 1 und 2 an molekularem Sauerstoff werden anfänglich zunächst noch weitgehend identisch gewählt. Anschließend liegt die Zuwachsrate des Mengenstroms 2 an molekularem Sauerstoff oberhalb derjenigen des Mengenstroms 1 an molekularem Sauerstoff, bevor die Zuwachsrate des Mengenstroms 1 an molekularem Sauerstoff diejenige des Mengenstroms 2 an molekularem Sauerstoff überwiegt und der Mengenstrom 2 an molekularem Sauerstoff hinter den Mengenstrom 1 an molekularem Sauerstoff zurückfällt. Insgesamt werden die Mengenströme 1, 2 an molekularem Sauerstoff (jeweils netto gerechnet) über das Betriebszeitpunktintervall t₀ < t < t_{R} im Vergleich zur Temperatur des Reaktionsgasgemischstroms beim Eintritt desselben in das in Strömungsrichtung erste Drittel der Menge M so erhöht, dass die Temperaturzuwachsrate von T₃* dabei im wesentlich stetig oberhalb der Temperaturzuwachsrate von T₁* liegt. Die Temperaturzuwachsrate von T₂* liegt dabei dagegen normalerweise anfänglich oberhalb der Temperaturzuwachsrate von T₃*, um im weiteren Betriebsverlauf unter die Temperaturzuwachsrate von T₃* zu fallen und sich der geringeren Zuwachsrate von T₁* immer mehr anzunähern.

In besonders einfacher Weise ist die ebenda beschriebene erfindungsgemäße Langzeitbetriebsweise in einem adiabat gestalteten Hordenreaktor, insbesondere in einem Katalysatorfestbetthordenreaktor, realisierbar. Erfindungsgemäß günstig weist der Katalysatorfestbetthordenreaktor drei, vorzugsweise identische, Katalysatorfestbetthorden (Katalysatorteilfestbetten) auf. Die Sauerstoffzwischeneinspeisung kann zwischen den Katalysatorfestbetthorden erfolgen. Die Dehydrierkatalysatorteilfestbetten können in Strömungsrichtung des Reaktionsgasgemischstroms axial hintereinander oder in den Ringspalten von zentrisch ineinandergestellten zylindrischen Gitterrosten räumlich nacheinander angeordnet sein. In besonders einfacher Weise wird dabei ein adiabat gestalteter Schachtofenreaktor eingesetzt, wie er z. B. in der deutschen Anmeldung Nr. 10 2006 015 235, der deutschen Anmeldung Nr. 10 2006 017 623, der deutschen Anmeldung Nr. 10 2006 029 790 und in der DE-A 10 2005 051 401 beschrieben ist.

Um bei der Sauerstoffzwischeneinspeisung die insgesamt zugeführten Mengenströme über die Betriebsdauer im wesentlichen konstant zu halten, enthält das zudosierte molekularen Sauerstoff enthaltende Gas anwendungstechnisch vorteilhaft Wasserdampf als inertes begleitendes Verdünnungsgas zugesetzt. Mit zunehmendem Sauerstoffmengestrom geht dann in vorteilhafter Weise ein abnehmender Wasserdampfmengenstrom einher, so dass der insgesamt jeweils zugeführte Mengenstrom an Sauerstoff enthaltendem Gas im wesentlichen konstant bleibt (über die Betriebsdauer).

Erfindungsgemäß vorteilhaft wird eine Zwischeneinspeisung eines molekularen Sauerstoff enthaltenden Gases so vorgenommen, dass bei homogener Vermischung desselben mit dem Reaktionsgasgemischstrom an der Zudosierungsstelle ein neues Reaktionsgasgemisch entsteht, in welchem das molare Verhältnis von enthaltenem molekularem Sauerstoff zu enthaltenem molekularem Wasserstoff ≤ 1 : 2 beträgt. Die Einhaltung dieser Rahmenbedingung gewährleistet eine erhöhte Zielproduktselektivität.

Es überrascht, dass die zur Realisierung einer erfindungsgemäßen Langzeitbetriebsweise erforderliche Temperaturlenkung trotz der vorgenannten Rahmenbedingung im wesentlichen ohne Verlust an Zielproduktselektivität auch auf dem Weg einer wie beschrieben anzuwendenden direkten Temperaturlenkung möglich ist, ohne in notwendiger Weise einer Zufuhr von externem molekularem Wasserstoff zu bedürfen. Ist im vorgenannten Zusammenhang ein erhöhter Gehalt an molekularem Wasserstoff erforderlich, kann zur Einstellung desselben anwendungstechnisch zweckmäßig auch die in den Schriften WO 03/076370 bzw. DE-A 102 11 275 beschriebene Schlaufenfahrweise angewendet werden, bei der man den der Reaktionszone RZ entnommenen Produktgasstrom in zwei Teilmengen identischer Zusammensetzung aufteilt und eine der beiden Teilmengen als Dehydrierkreisgas zur Beschickung der Reaktionszone RZ in selbige zurückführt. Eine heterogen katalysierte partielle Dehydrierung mit Schlaufenfahrweise offenbart auch die WO 2006/050957. Bei Bedarf kann dem Reaktionsgasgemischstrom beim erfindungsgemäßen Verfahren aber auch jederzeit externer molekularer Wasserstoff zugeführt werden. Insbesondere dann, wenn auf eine erhöhte Standzeit des Katalysatorgesamtbetts Wert gelegt wird, ist es erfindungsgemäß vorteilhaft, bereits dem der Reaktionszone RZ zugeführten Reaktionsgasgemischstrom molekularen Wasserstoff zuzusetzen. Dies gilt vor allem dann, wenn der vorgenannte Reaktionsgasgemischstrom molekularen Sauerstoff enthält. Dies ist in der Regel insbesondere dann der Fall, wenn sich an das erfindungsgemäße Verfahren ein Verfahren der heterogen katalysierten Partialoxidation von erzeugtem dehydriertem Kohlenwasserstoff (z. B. Propylen zu Acrolein und/oder Acrylsäure) in Begleitung von nicht umgesetztem zu dehydrierendem Kohlenwasserstoff (z. B. Propan) als Inertgas in der Partialoxidation anschließt, und man dabei wie folgt verfährt:
Zunächst wird man den aus der Reaktionszone RZ entnommenen Produktgasstrom als solchen oder nach Abtrennung wenigstens einer Teilmenge seiner vom dehydrierten Kohlenwasserstoff (z. B. Propylen) und vom (nicht umgesetzten) zu dehydrierenden Kohlenwasserstoff (z. B. Propan) verschiedenen Bestandteile (z. B. H₂, H₂O, N₂, etc.) zur Beschickung wenigstens eines Oxidationsreaktors verwenden, und den im Beschickungsgasgemisch enthaltenen dehydrierten Kohlenwasserstoff (z. B. Propylen) einer selektiven heterogen katalysierten partiellen Gasphasenoxidation mit molekularem Sauerstoff zu einem das (Partialoxidationsprodukt) Zielprodukt (z. B. Acrolein, oder Acrylsäure, oder deren Gemisch), sowie in der Regel nicht umgesetzten zu dehydrierenden Kohlenwasserstoff (z. B. Propan), überschüssigen molekularen Sauerstoff und gegebenenfalls nicht umgesetzten dehydrierten Kohlenwasserstoff (z. B. Propylen) enthaltenden Partialoxidationsproduktgasgemisch unterwerfen.

In einer nachfolgenden Trennzone wird man im Partialoxidationsproduktgasgemisch enthaltenes Zielprodukt (z. B. Acrolein, oder Acrylsäure, oder deren Gemisch) abtrennen, und von dem dabei verbleibenden, nicht umgesetzten zu dehydrierenden Kohlenwasserstoff (z. B. Propan), molekularen Sauerstoff sowie gegebenenfalls nicht umgesetzten dehydrierten Kohlenwasserstoff (z. B. Propylen) enthaltenden Restgas wenigstens eine nicht umgesetzten zu dehydrierenden Kohlenwasserstoff (z. B. Propan) und meistens nicht umgesetzten molekularen Sauerstoff sowie gegebenenfalls nicht umgesetzten dehydrierten Kohlenwasserstoff (z. B. Propylen) enthaltende Teilmenge als Partialoxidationskreisgas in das erfindungsgemäße Verfahren rückführen (in der Regel als Bestandteil des der Reaktionszone RZ zugeführten Reaktionsgasgemischstroms, der die Ausgangsmenge KW an zu dehydrierendem Kohlenwasserstoff enthält).

Handelt es sich beim erfindungsgemäßen Verfahren (aber auch sonst) z. B. um eine heterogen katalysierte partielle Dehydrierung von Propan zu Propylen, und bei der sich anschließenden Partialoxidation um diejenige von Propylen zu Acrolein, oder zu Acrylsäure, oder zu deren Gemisch, kann der der Reaktionszone RZ zugeführte Reaktionsgasgemischstrom als wesentliche Gehalte z. B. enthalten:

| | |
|---|---|
| Propylen | ≥ 0 bis 20 bzw. bis 10, häufig 0 bis 6 Vol.-%, |
| Acrolein | ≥ 0 bis 1, vielfach 0 bis 0,5, häufig 0 bis 0,25 Vol.-%, vorzugsweise 0 bis 0,05 Vol.-%, |
| Acrylsäure | ≥ 0 bis 0,25 (oder bis 0,4), vielfach 0 bis 0,05, häufig 0 bis 0,03 Vol.-%, |
| COₓ | ≥ 0 bis 20 bzw. bis 5, vielfach 0 bis 3, häufig 0 bis 2 Vol.-%, |
| Propan | 5 bis 50, vorzugsweise 20 bis 40 Vol.-%, |
| Stickstoff | 20 bzw. 30 bis 80, vorzugsweise 50 bis 70 Vol.-%, |
| Sauerstoff | ≥ 0 bis 5, vorzugsweise 1,0 bis 2,0 Vol.-%, |
| H₂O | ≥ 0 bis 20, vorzugsweise 5,0 bis 10,0 Vol.-%, und |
| H₂ | ≥ 0, häufig ≥ 0,01, oft ≥ 0,05 bis 10, vorzugsweise 1 bis 5 Vol.-%. |

In geringer Menge (etwa vergleichbar den möglichen Acrylsäuregehalten) kann auch Essigsäure enthalten sein.

Üblicherweise erfolgt die Abtrennung von Zielprodukt (z. B. Acrylsäure) aus dem Partialoxidationsproduktgasgemisch dadurch, dass man das Zielprodukt (z. B. die Acrylsäure) in die kondensierte Phase überführt. Dies kann durch absorptive und/oder kondensative (Kühlen) Maßnahmen erfolgen. Als Absorptionsmittel kommen im Fall von Acrylsäure als Zielprodukt z. B. Wasser, wässrige Lösungen oder hochsiedende (T_{siede} von Lösungsmittel > T_{Siede} von Acrylsäure bei 1 atm), insbesondere hydrophobe, organische Lösungsmittel in Betracht. Besonders bevorzugt erfolgt das Überführen in die kondensierte Phase im Fall der Acrylsäure durch fraktionierende Kondensation des Partialoxidationsproduktgasgemisches. Vorzugsweise erfolgt die absorptive und/oder kondensative Überführung der Acrylsäure aus dem Partialoxidationsproduktgasgemisch in die kondensative Phase in trennwirksame Einbauten enthaltenden Kolonnen, in denen das Partialoxidationsproduktgasgemisch normalerweise von unten nach oben aufsteigend geführt wird. Das Absorptionsmittel wird in der Regel am Kolonnenkopf aufgegeben, an dem das Restgas normalerweise aus der Kolonne entlassen wird.

Die weitere Abtrennung der Acrylsäure aus der kondensierten Phase erfolgt in der Regel in der gewünschten Reinheit unter Anwendung wenigstens eines thermischen Trennverfahrens. Darunter werden solche Verfahren verstanden, bei denen wenigstens zwei voneinander verschiedene stoffliche Phasen (z. B. flüssig/flüssig; gasförmig/flüssig; fest/flüssig; gasförmig/fest etc.) erzeugt und miteinander in Kontakt gebracht werden. Aufgrund der zwischen den Phasen bestehenden Gradienten findet zwischen denselben ein Wärme- und Stoffaustausch statt, der letztlich die gewünschte Auftrennung (Abtrennung) bedingt. Die Bezeichnung "thermische Trennverfahren" reflektiert dabei, dass es entweder des Entzuges oder der Zufuhr von Wärme bedarf, um die Ausbildung der stofflichen Phasen zu erzeugen und/oder dass der Entzug oder die Zufuhr von thermischer Energie den Stoffaustausch begünstigt bzw. aufrechterhält. Erfindungsgemäß bevorzugt umfasst das wenigstens eine thermische Trennverfahren wenigstens eine kristallisative Abtrennung aus flüssiger Phase. Erfindungsgemäß zweckmäßig ist die wenigstens eine kristallisative Abtrennung der Acrylsäure eine Suspensionskristallisation und mit Vorteil wird das Suspensionskristallisat mit geschmolzenem, zuvor abgetrenntem und gewaschenem Kristallisat in einer Waschkolonne (eine gravimetrische, oder eine mechanische, oder eine hydraulische Waschkolonne; letztere ist erfindungsgemäß bevorzugt) gewaschen. Im übrigen kommen als thermische Trennverfahren z. B. extraktive, desorptive, kristallisative, rektifikative, azeotrop-destillative, azeotrop-rektifikative, destillative und/oder Strippverfahren in Betracht. In der Regel wird man zur Gewinnung von reiner Acrylsäure Kombinationen von verschiedenen der genannten thermischen Trennverfahren anwenden.

An die beschriebene Abtrennung der Acrylsäure kann sich ein Verfahren der radikalischen Polymerisation (insbesondere zur Herstellung von Wasser superabsorbierenden Polyacrylsäuren und/oder deren teil- bzw. vollneutralisierten Alkali(vorzugsweise Na)metallsalzen) anschließen, in welchem abgetrennte Acrylsäure zur Herstellung von Polymerisaten radikalisch einpolymerisiert wird.

Auch kann sich an die beschriebene Abtrennung der Acrylsäure ein Verfahren zur Herstellung von Acrylsäureestern anschließen, in welchem abgetrennte Acrylsäure mit Alkoholen (vorzugsweise Alkanolen, besonders bevorzugt C₁- bis C₁₂-Alkanolen) (in der Regel säurekatalysiert) verestert wird.

An das Verfahren der Veresterung kann sich wiederum ein Verfahren der radikalischen Polymerisation anschließen, in welchem so hergestellter Acrylsäureester einpolymerisiert wird.

Sieht man von der Besonderheit der erfindungsgemäßen Langzeitbetriebsweise ab, sind erfindungsgemäße Verfahren zur Herstellung von Propylen aus Propan als Propylenquelle für Partialoxidationen desselben zur Herstellung von Acrolein und/oder Acrylsäure vorbekannt, einschließlich einer Kreisgasführung von Oxidationskreisgas und gegebenenfalls Dehydrierkreisgas. Beispielsweise findet man solche mehrstufigen Verfahren beschrieben in den Schriften DE-A 10 2005 022 798, Deutsches Aktenzeichen 10 2006 024 901.1, DE-A 102 46 119, DE-A 102 45 585, DE-A 10 2005 049 699, DE-A 10 2004 032 129, DE-A 10 2005 013 039, DE-A 10 2005 010 111, DE-A 10 2005 009 891, DE-A 102 11 275, EP-A 117 146, US 3,161,670, DE-A 33 13 573, WO 01/96270, DE-A 103 16 039, DE-A 10 2005 009 885, DE-A 10 2005 052 923, DE-A 10 2005 057 197, WO 03/076370, DE-A 102 45 585, DE-A 22 13 573, US 3,161,670 und dem in diesen Schriften zitierten Stand der Technik. Die DE-A 102 19 686 offenbart die entsprechende Vorgehensweise im Fall einer Herstellung von Methacrolein und/oder Methacrylsäure.

Absorptive und/oder kondensative Verfahren zur Überführung von Acrylsäure aus einem Partialoxidationsproduktgasgemisch in die kondensierte Phase finden sich ebenfalls ausführlich im Stand der Technik beschrieben. Angeführt seien die Schriften DE-A 103 36 386, DE-A 196 31 645, DE-A 195 01 325, EP-A 982 289, DE-A 198 38 845, WO 02/076917, EP-A 695 736, EP-A 778 225, EP-A 1 041 062, EP-A 982 287, EP-A 982 288, US-A 2004/0242826, EP-A 792 867, EP-A 784 046, EP-A 695 736 (insbesondere absorptive) sowie WO 04/035514, DE-A 199 24 532, DE-A 198 14 387, DE-A 197 40 253, DE-A 197 40 252 und DE-A 196 27 847 (insbesondere kondensative).

Ferner finden sich solche absorptiven und/oder kondensativen Abtrennungen von Acrylsäure aus Partialoxidationsproduktgasgemischen auch beschrieben in den Schriften EP-A 1 338 533, EP-A 1 388 532, DE-A 102 35 847, WO 98/01415, EP-A 1 015 411, EP-A 1 015 410, WO 99/50219, WO 00/53560, WO 02/09839, DE-A 102 35 847, WO 03/041833, DE-A 102 23 058, DE-A 102 43 625, DE-A 103 36 386, EP-A 854 129, US 4,317,926, DE-A 198 37 520, DE-A 196 06 877, DE-A 195 01 325, DE-A 102 47 240, DE-A 197 40 253, EP-A 695 736, EP-A 1 041 062, EP-A 117 146, DE-A 43 08 087, DE-A 43 35 172, DE-A 44 36 243, DE-A 103 32 758 sowie DE-A 199 24 533. Grundsätzlich kann dabei aber auch wie in der DE-A 103 36 386, DE-A 101 15 277, DE-A 196 06 877, EP-A 920 408, EP-A 1 068 174, EP-A 1 066 239, EP-A 1 066 240, WO 00/53560, WO 00/53561, DE-A 100 53 086, WO 01/96271, DE-A 10 2004 032 129, WO 04/063138, WO 04/35514, DE-A 102 43 625 und DE-A 102 35 847 beschrieben vorgegangen werden.

Handelt es sich beim zu dehydrierenden Kohlenwasserstoff für das erfindungsgemäße Verfahren um Propan, wird man selbigen vorzugsweise als Bestandteil von Roh-Propan gemäß der Lehre der DE-A 102 45 585 dem in die Reaktionszone RZ zugeführten Reaktionsgasgemischstrom zuführen.

Generell wird der der Reaktionszone RZ zugeführte Reaktionsgasgemischstrom wenigstens zu 5 Vol.-% an zu dehydrierendem Kohlenwasserstoff enthalten. Häufig liegt dieser Volumenanteil bei entsprechend bezogenen Werten von ≥ 10 Vol.-%, oft ≥ 15 Vol.-% und meist ≥ 20 Vol.-% bzw. ≥ 25 Vol.-%, oder ≥ 30 Vol.-%. In der Regel liegt dieser Volumenanteil jedoch bei in gleicher Weise bezogenen Werten von ≤ 90 Vol.-%, meist ≤ 80 Vol.-% und oft ≤ 70 Vol.-%. Vorstehende Angaben gelten insbesondere im Fall von Propan als zu dehydrierendem Kohlenwasserstoff und Propylen als dehydriertem Kohlenwasserstoff. Selbstredend treffen sie aber auch dann zu, wenn iso-Butan der zu dehydrierende Kohlenwasserstoff und iso-Buten der dehydrierte Kohlenwasserstoff ist.

Daneben kann der vorgenannte Reaktionsgasgemischstrom enthalten:
a) N₂ und H₂O;
b) N₂, O₂ und H₂O;
c) N₂, O₂, H₂O und H₂;
d) N₂, O₂, H₂O, H₂ und CO₂;
e) N₂, O₂, H₂O, H₂, CO₂ und CO.

In günstigen Fällen ist der beim erfindungsgemäßen Verfahren der Reaktionszone RZ zugeführte Reaktionsgasgemischstrom durch Zusammenführen von z. B. Roh-Propan, molekularem Wasserstoff und Partialoxidationskreisgas entstanden. Anwendungstechnisch zweckmäßig führt man dabei vorab das Roh-Propan und den molekularen Wasserstoff zusammen, und erwärmt dieses Gemisch auf Reaktionstemperatur (dies ist üblicherweise eine Temperatur von ≥ 300 °C, vorzugsweise ≥ 350 °C und vielfach ≥ 400 °C). Das Partialoxidationskreisgas wird ebenfalls auf Reaktionstemperatur erwärmt. Beide Erwärmungen können durch indirekten Wärmeaustausch mit heißem Produktgasgemisch des erfindungsgemäßen Verfahrens erfolgen. Anschließend führt man die beiden vorgewärmten Gasströme zum der Reaktionszone RZ zuzuführenden Reaktionsgasgemischstrom zusammen. Eine im Rahmen des erfindungsgemäßen Langzeitbetriebs gegebenenfalls erforderliche Erhöhung der Temperatur des der Reaktionszone RZ zugeführten Reaktionsgasgemischstroms kann z. B. durch Intensivierung des indirekten Wärmeaustauschs bewirkt werden.

Abschließend sei nochmals darauf hingewiesen, dass zum Zweck der erfindungsgemäßen Langzeitbetriebsweise die Maßnahmen der direkten sowie indirekten Temperaturlenkung jeweils alleine oder aber auch kombiniert angewendet werden können. Besonders einfach ist das erfindungsgemäße Verfahren in Hordenreaktoren umsetzbar, deren Horden adiabat gestaltet sind, und wo zwischen zwei Horden die jeweils erforderlichen Lenkungs- und Mengenstromvariationen gegebenenfalls zugeführter Dehydrierhilfsgase ergriffen werden können. Die Hordenanzahl ist dabei in einem weiten Bereich variabel. Anwendungstechnisch zweckmäßig sind die Katalysatorhorden auf identische Art und Weise mit Dehydrierkatalysator beschickt. In günstiger Weise beträgt die Hordenanzahl drei, oder ein ganzzahliges Vielfaches (z. B. sechs, neun, zwölf) davon. Anstelle eines Hordenreaktors kann jedoch auch eine Hintereinanderschaltung einer der Hordenzahl entsprechenden Anzahl von adiabaten Einzelreaktoren angewendet und die Lenkungsmaßnahmen zwischen solchen Reaktoren angewendet werden. Grundsätzlich kann die erfindungsgemäße Verfahrensweise aber auch an einem einzigen in Strömungsrichtung ohne Unterbrechung durchgehenden Katalysatorbett angewendet werden.

Generell werden mit der erfindungsgemäßen Verfahrensweise bei einer heterogen katalysierten partiellen Dehydrierung Betriebszeiten vorab einer nächsten Regenerierung des Katalysatorbetts erzielt, die wenigstens 20 h, vielfach jedoch bis zu 100 h und mehr betragen.

### Ausführungsbeispiel und Vergleichsbeispiel

### a) Beschreibung des Dehydrierreaktors

Als Dehydrierreaktor wurde ein Stahlrohr (Edelstahl der DIN Werkstoffnummer 1.4841) der Länge 2 070 mm, der Wanddicke 1 mm und des Innendurchmessers 36,4 mm verwendet. Der Rohrreaktor wurde vom Reaktionsgasgemischstrom von oben nach unten durchströmt. Ins untere Ende des Rohrreaktors ragte ein 115 mm hoher Kontaktstuhl aus demselben Edelstahl, der das Katalysatorgesamtfestbett (bestehend aus drei Katalysatorteilfestbetten von ca. identischem Schüttgewicht) wie folgt von oben nach unten strukturiert trug:

| | |
|---|---|
| 790 mm | Schüttlänge aus (inerten) Steatitkugeln (Durchmesser 4 - 5 mm) des Steatits C-220 der Fa. CeramTec (interne Aufheizschüttung); |
| 195 mm | Schüttlänge Dehydrierkatalysator (Pt/Sn-Legierung, die mit den Elementen Cs, K und La in oxidischer Form promoviert war und die auf die äußere und innere Oberfläche von ZrO₂ • Si02 Mischoxidträgerstränglingen (mittlere Länge (gaußverteilt im Bereich von 3 mm bis 12 mm mit Maximum bei ca. 6 mm) : 6 mm, Durchmesser: 2 mm) in der Elementstöchiometrie (Massenverhältnis einschließlich Träger) |
| | Pt_{0,3}Sn_{0,6}La_{3,0}Cs_{0,5}K_{0,2}(ZrO₂)_{88,3}(SiO₂)_{7,1} aufgebracht war (Katalysatorvorläuferherstellung und Aktivierung zum aktiven Katalysator wie in Beispiel 4 der DE-A 102 19 879); |
| 145 mm | Schüttlänge aus Steatitkugeln (Durchmesser 4 - 5 mm) des Steatits C-220 der Fa. CeramTec; |
| 190 mm | Schüttlänge des vorgenannten Dehydrierkatalysators; |
| 145 mm | Schüttlänge aus Steatitkugeln (Durchmesser 4 - 5 mm) des Steatits C-220 der Fa. CeramTec; |
| 195 mm | Schüttlänge des vorgenannten Dehydrierkatalysators; |
| 20 mm | Schüttlänge aus Steatitkugeln (Durchmesser 1,5 - 2,5 mm) des Steatits C-220 der Fa. CeramTec; und |
| 210 mm | Schüttlänge aus Steatitkugeln (Durchmesser 4 - 5 mm) des Steatits C-220 der Fa. CeramTec. |

Oberhalb der internen Aufheizschüttung war das Stahlrohr leer.

Der Rohrreaktor war außen im Sinne einer Vorheizstrecke auf der Länge der obersten 400 mm der Aufheizschüttung von oben nach unten (zum Kontaktstuhl hin) in zwei, eine Gleichverteilung der zugeführten Wärmemenge gewährleistende, Halbschalen aus Kupfer (Schalendicke = 25 mm) eingelegt, die mittels eines sie im vollen Umfang umgebenden Heizbandes elektrisch beheizt wurden. Um das Heizband befand sich eine Wicklung aus thermischem Isoliermaterial.
Von unten nach oben (kurz unterhalb der Kontaktstuhlauflagefläche beginnend) war der Rohrreaktor auf einer Länge von 1 530 mm in zwei Paare von thermisch isolierend wirkenden Halbschalen (Dicke einer Halbschale = 25 mm) aus MPS-Super G der Fa. Microtherm in DE, die um 90° gegeneinander versetzt übereinander angebracht waren, eingebracht. Die isolierend wirkenden Halbschalen waren ihrerseits von einer zylindrischen Einhüllenden aus Edelstahl (Außendurchmesser = 168 mm, Innendurchmesser = 154 mm) umgeben, um die herum zum Zweck der Begleitheizung ein Strahlungsofen (Länge = 1 600 mm) angeordnet war. Auf diese Weise konnte auf der adiabaten Strecke der Wärmefluss aus der Umgebung in das Reaktionsrohr hinein und aus dem Reaktionsrohr heraus in die Umgebung minimiert werden.

In das Reaktionsrohr war mittig (zentral) zusätzlich eine 2 500 mm lange Thermohülse eingeführt (Außendurchmesser = 6 mm; Innendurchmesser = 4 mm), in die ein Mehrfach-Thermoelement (vom unteren Reaktorende nach oben alle 8 cm insgesamt 14 Messstellen, Dicke 3,2 mm) eingeführt war.

Zusätzlich waren zwei Lanzenpaare in den Rohrreaktor eingeführt. Das eine Paar war von unten und das andere Paar von oben in den Rohrreaktor eingeführt. Die Lanzen eines Paares wurden jeweils aneinanderliegend zwischen Thermohülse und Reaktorinnenwand so geführt, dass ihre Positionierung über den Rohrquerschnitt zwischen Thermohülse und Reaktorinnenwand mittig war. In eine Rohrquerschnittsebene projeziert standen sich die beiden Paare auf einem Rohrdurchmesser gegenüber. Der Außendurchmesser einer aus Edelstahl der DIN Werkstoffnummer 1.4841 gefertigten Lanze betrug 3,17 mm und ihr Innendurchmesser lag bei 2,17 mm. Die Länge der Lanzen eines Paares war unterschiedlich. Durch jeweils eine der beiden Lanzen eines Lanzenpaares wurde Luft zudosiert und durch die jeweils andere Lanze eines Lanzenpaares wurde Reaktionsgasgemisch zu Analysezwecken entnommen. Die in Strömungsrichtung erste Analysenlanze (LI) war mit ihrer Öffnung 20 mm hinter dem in Strömungsrichtung ersten Katalysatorteilfestbett platziert. Die zugehörige Zudosierungslanze (ZI) war mit ihrer Öffnung 100 mm vor dem in Strömungsrichtung zweiten Katalysatorteilfestbett platziert. Die in Strömungsrichtung zweite Analysenlanze (LII) war mit ihrer Öffnung 20 mm hinter dem in Strömungsrichtung zweiten Katalysatorteilfestbett platziert. Die zugehörige Zudosierungslanze (ZII) war mit ihrer Öffnung 100 mm vor dem in Strömungsrichtung dritten Katalysatorteilfestbett platziert.

Vor dem Rohrreaktor war ein mit Steatitkugeln (aus Steatit C-220 der Fa. CeramTec, Durchmesser 4 - 5 mm) gefülltes Stahlrohr der Länge 1 300 mm als Erhitzer geschaltet. In ihm wurde der Reaktionsgasgemischstrom auf seine Eintrittstemperatur in den Rohrreaktor vorgeheizt und gleichzeitig ideal durchmischt. Zu diesem Zweck wurde das Erhitzerrohr (Edelstahl der DIN Werkstoffnummer 1.4841, Wanddicke 3,6 mm, Innendurchmesser 53,1 mm) auf einer rohrmittigen Länge von 1 200 mm mittels um sie angelegter Heizmanschetten der Fa. Horst, DE-Heidelberg elektrisch beheizt. Die Verbindung zwischen Erhitzer und Rohrreaktor wurde durch ein thermisch beheiztes und mit üblichen Wärmedämmmaterialien thermisch isoliertes Edelstahlrohr (Edelstahl der DIN Werkstoffnummer 1.4841, Außendurchmesser 14 mm, Innendurchmesser 10 mm, Länge 300 mm) bewerkstelligt.

### b) Beginn eines erfindungsgemäßen Langzeitbetriebs (Betriebszeitpunkt to)

Das Katalysatorgesamtbett war mit frischem Dehydrierkatalysator beschickt. Der dem Katalysatorgesamtfestbett zugeführte Reaktionsgasgemischstrom war ein Gemisch aus Roh-Propan, Wasserdampf und Partialoxidationskreisgas einer heterogen katalysierten zweistufigen Partialoxidation des in der Dehydrierung erzeugten Propylens zu Acrylsäure. Aus dem Produktgasgemisch der Dehydrierung wurden die von Propan und Propylen verschiedenen Bestandteile wie im Vergleichsbeispiel 1 der deutschen Anmeldung Nr. 10 2005 013 039 beschrieben absorptiv/desorptiv (Strippen mit Luft) abgetrennt. Die Partialoxidation des Propylens zu Acrylsäure erfolgte ebenfalls wie in der deutschen Anmeldung Nr. 10 2005 013 039 beschrieben. Das gleiche gilt für die Bildung des Partialoxidationskreisgases.

Der Reaktionsgasgemischstrom wurde wie in der deutschen Anmeldung Nr. 10 2005 013 039 beschrieben mit einer Austrittstemperatur von etwa 200 °C in einem Verdampfer erzeugt und von diesem ausgehend dem Erhitzer zugeführt (die Anbindung des Verdampfers an den Erhitzer war wie diejenige des Erhitzers an den Rohrreaktor gestaltet).

Die Beheizung des Erhitzers war so geregelt, dass der vom Verdampfer in den Erhitzer geleitete Reaktionsgasgemischstrom diesen mit einer Temperatur von etwa 400 °C verließ. Dann wurde der Reaktionsgasgemischstrom in den Rohrreaktor geführt und in der Vorheizstrecke desselben weiter erhitzt und schließlich durch den Rohrreaktor geführt. Über die Zudosierungslanzen ZI und ZII wurden jeweils etwa 30 NI/h Luft zudosiert, die am Eingang in die jeweilige Lanze eine Temperatur von 25 °C aufwies. Nach einer Betriebszeit von to = 1 h erreichte der Verfahrensbetrieb im wesentlichen erstmals seinen (quasi) stationären Betriebszustand.

Die Stoffgehalte des dem Rohrreaktor zugeführten Reaktionsgasgemischstroms (alle Gaszusammensetzungsangaben in dieser Schrift basieren stets auf gaschromatographischer Analyse) von 2 807 Nl/h sowie die Stoffgehalte des Reaktionsgasgemischstroms beim Verlassen des in Strömungsrichtung ersten, zweiten sowie dritten Katalysatorteilfestbetts waren dabei wie in der Tabelle 1 angegeben.

**Tabelle 1**

| | Reaktoreingang | nach 1. Teilbett | nach 2. Teilbett | nach 3. Teilbett |
|---|---|---|---|---|
| Vol.ppm Acrolein | 235 | < 2 | < 2 | < 2 |
| Vol.ppm Acrylsäure | 344 | < 2 | < 2 | < 2 |
| Vol.ppm Essigsäure | 170 | < 2 | < 2 | < 2 |
| Vol.-% Wasserstoff | 0,05 | 2,42 | 3,15 | 4,37 |
| Vol.-% Sauerstoff | 2,93 | < 2 | < 2 | < 2 |
| Vol.-% Stickstoff | 53 | 51 | 51 | 51 |
| Vol.-% Kohlenmonoxid | 0,36 | 0,15 | 0,16 | 0,18 |
| Vol.-% Kohlendioxid | 1,29 | 2,20 | 2,53 | 2,48 |
| Vol.ppm Methan | 39 | 175 | 342 | 386 |
| Vol.ppm Ethan | 305 | 313 | 357 | 447 |
| Vol.ppm Ethen | 328 | 397 | 565 | 599 |
| Vol-ppm Acetylen | 2 | 14 | 25 | 26 |
| Vol.-% Propan | 35,4 | 32,5 | 29,6 | 27,8 |
| Vol.ppm Cyclopropan | 200 | 85 | 35 | 19 |
| Vol.-% Propen | 0,34 | 4,92 | 6,90 | 7,33 |
| Vol.ppm Propadien | < 2 | < 2 | < 2 | < 2 |
| Vol.ppm Propin | < 2 | 28 | 50 | 52 |
| Vol.ppm iso-Butan | 71 | 63 | 57 | 51 |
| Vol.ppm n-Butan | 3 | 3 | 3 | 3 |
| Vol.ppm trans-Buten-2 | 2 | 2 | 2 | 3 |
| Vol.ppm Buten-1 | < 2 | < 2 | 2 | 3 |
| Vol.ppm iso-Buten | 5 | 7 | 12 | 17 |
| Vol.ppm cis-Buten-2 | < 2 | < 2 | 2 | 2 |
| Vol.pmm Butadien-1.3 | 7 | 2 | 2 | 2 |
| Vol.ppm sonstige C₂-C₄-Kohlenwasserstoffe | < 2 | < 2 | < 2 | < 2 |
| Vol.ppm Benzol | 83 | 69 | 69 | 78 |
| Vol.ppm Restsumme C₅-Kohlenwasserstoffe | 34 | 9 | 10 | 17 |
| Vol.ppm Reststumme C₆-Kohlenwasserstoffe | 8 | 7 | 7 | 5 |

| | | | | |
|---|---|---|---|---|
| Restmenge bis zu 100 Vol.-% = jeweils Wasser | | | | |

Beim Durchgang des Reaktionsgasgemischstroms durch das Katalysatorgesamtbett wurden insgesamt G = (A + B + C) mol-% der in diesem enthaltenen molaren Ausgangsmenge KW an zu dehydrierendem Kohlenwasserstoff zu dehydriertem Kohlenwasserstoff dehydriert. A ist dabei der Beitrag des in Strömungsrichtung ersten Katalysatorteilfestbetts, B ist der Beitrag des in Strömungsrichtung zweiten Katalysatorteilfestbetts und C ist der Beitrag des in Strömungsrichtung dritten Katalysatorteilfestbetts.

Zum Betriebszeitpunkt to lauteten die numerischen Werte für G, A, B und C wie folgt:
A = 12 mol-%,
B = 5,4 mol-%,
C = 1,8 mol-%, und
G = 19,2 mol-%,
jeweils bezogen auf KW.

Die Temperatur T₁ des Reaktionsgasgemischstroms in Strömungsrichtung unmittelbar hinter der internen Aufheizschüttung im Rohrreaktor betrug zum Betriebszeitpunkt to = 408 °C. Die zum Betriebszeitpunkt t₀ über ZI und ZII zudosierten Luftmengenströme MI und MII waren jeweils ca. 33 Nl/h.

### c) Durchführung eines erfindungsgemäßen Langzeitbetriebs

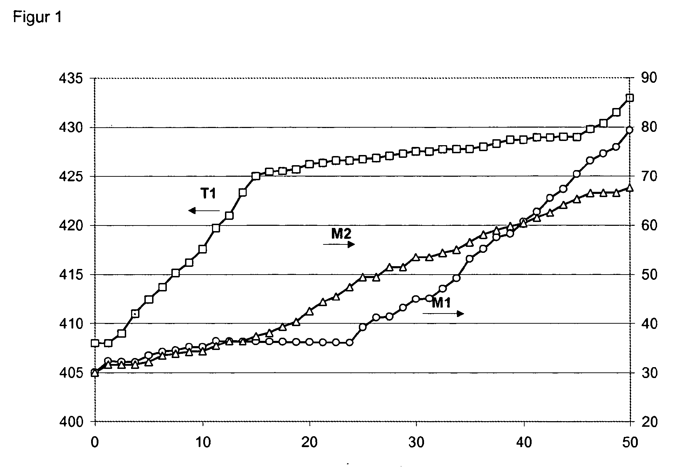

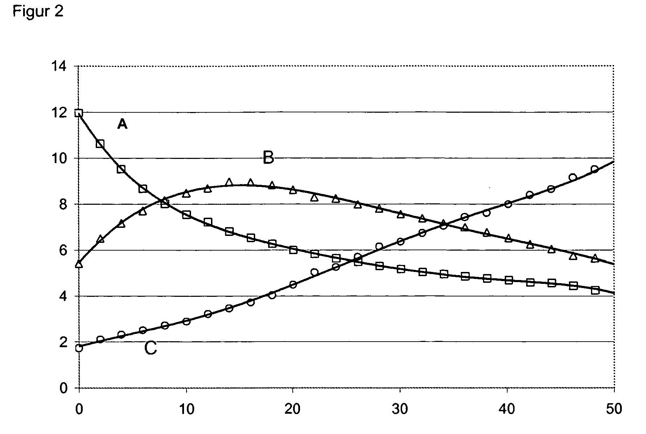

Um einer mit zunehmender Betriebsdauer einhergehenden Deaktivierung des Katalysatorgesamtfestbetts entgegenzuwirken, wurden sowohl T₁ als auch MI (in Fig. 1 = M1) und MII (in Fig. 1 = M2) mit zunehmender Betriebsdauer wie in Figur 1 (anhand der erhobenen Messpunkte) gezeigt sukzessive angehoben (linke Ordinate = T₁ in °C; rechte Ordinate = Luftmengenstrom in Nl/h; der Nullpunkt wurde in to gelegt; die Abszisse zeigt t in h). Auf diese Art und Weise gelang es über das Betriebszeitpunktintervall 0 h = t₀ < t ≤ 50 h den Wert für G im Intervall G = 19,2 ± 0,2 mol-% stabil zu halten. Den dabei resultierenden zeitlichen Verlauf (die Abszisse zeigt t in h) der erhobenen Umsatzanteile A, B, C (mol-% von KW als Ordinate) im Betriebszeitpunktintervall zeigt Figur 2.

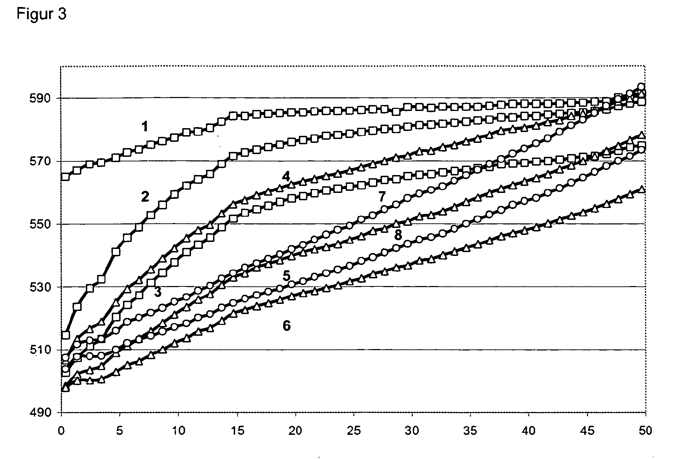

Den dabei resultierenden zeitlichen Verlauf der Temperatur des Reaktionsgasgemischstroms kurz hinter dem Eingang (die maximale Temperatur im Teilbett, Bezugsziffer 1), in der Mitte (Bezugsziffer 2) und am Ausgang (Bezugsziffer 3) des in Strömungsrichtung ersten Katalysatorteilfestbetts zeigt (anhand der erhobenen Messwerte) die Figur 3 (die Abszisse zeigt wieder t in h, der Nullpunkt wurde in t₀ gelegt, und die Ordinate zeigt T in °C) ebenso, wie den zeitlichen Verlauf der Temperatur des Reaktionsgasgemischstroms kurz hinter dem Eingang (die maximale Temperatur im Teilbett, Bezugsziffer 4), in der Mitte (Bezugsziffer 5) und am Ausgang (Bezugsziffer 6) des in Strömungsrichtung zweiten Katalysatorteilfestbetts bzw. des in Strömungsrichtung dritten Katalysatorteilfestbetts (Bezugsziffer 7 = die maximale Temperatur im dritten Katalysatorteilfestbett kurz hinter dem Eingang ins selbige, Bezugsziffer 8 = am Ausgang des dritten Katalysatorteilfestbetts).

Anschließend wurde das Verfahren unterbrochen, und das Katalysatorgesamtfestbett wie in der DE-A 100 28 582 beschrieben regeneriert. Danach wurde das Verfahren wieder aufgenommen. Nach einer Betriebsdauer von ca. 1 h war der (quasi) stationäre Betriebszustand wieder erreicht. Unter den ursprünglichen Betriebsbedingungen wurde dabei der ursprüngliche Wert für G wieder erzielt. Bis zur nächsten Regenerierung wird das Verfahren im erfindungsgemäßen Langzeitbetrieb weiterbetrieben u.s.w..

### d) Durchführung eines nicht erfindungsgemäßen Langzeitbetriebs

Die Inbetriebnahme erfolgte in identischer Weise wie in a), b) beschrieben. Um der mit zunehmender Betriebsdauer einhergehenden Deaktivierung des Katalysatorgesamtfestbetts entgegenzuwirken, wurden T₁ und die Luftmengenströme MI und MII mit zunehmender Betriebsdauer jeweils so angehoben, dass die Dehydrierumsatzbeiträge A, B, C und mit diesen deren Summe G stabil blieben.

Nach einer Betriebszeit von 17 h war auf der Grundlage dieser Langzeitbetriebsweise der Wert für G im Intervall G = 19,2 ± 1 mol-% nicht mehr aufrechtzuerhalten. Deshalb wurde das Verfahren unterbrochen, und das Katalysatorgesamtfestbett wie in der DE-A 100 28 582 beschrieben regeneriert. Anschließend wurde das Verfahren wieder aufgenommen. Unter den ursprünglichen Betriebsbedingungen konnte dabei der ursprüngliche Wert für G nicht mehr erreicht werden.

Beispiel und Vergleichsbeispiel können selbstredend in völlig äquivalenter Weise auch in einem Hordenfestbettreaktor, der drei identisch ausgeführte Festbetthorden aufweist, durchgeführt werden. Die Luftzufuhr erfolgt dann zwischen der in Strömungsrichtung 1./2. sowie 2./3. Horde.

US Provisional Patent Application No. 60/833776, eingereicht am 28.07.2006, ist eingefügt in die vorliegende Anmeldung durch Literaturhinweis.
Im Hinblick auf die oben genannten Lehren sind zahlreiche Änderungen und Abweichungen von der vorliegenden Erfindung möglich.
Man kann deshalb davon ausgehen, dass die Erfindung, im Rahmen der beigefügten Ansprüche, anders als hierin spezifisch beschrieben, ausgeführt werden kann.

## Patentansprüche

1. Ein Verfahren zum Langzeitbetrieb einer kontinuierlich betriebenen heterogen katalysierten partiellen Dehydrierung eines zu dehydrierenden Kohlenwasserstoffs zu einem dehydrierten Kohlenwasserstoff, bei dem man zum Zweck der heterogen katalysierten partiellen Dehydrierung des zu dehydrierenden Kohlenwasserstoffs einen diesen in einer molaren Ausgangsmenge KW enthaltenden Reaktionsgasgemischstrom so mit erhöhter Temperatur durch ein in einer Reaktionszone RZ befindliches Katalysatorgesamtbett führt, das aus mehreren in Strömungsrichtung des Reaktionsgasgemischstroms hintereinander angeordneten Katalysatorteilbetten bestehen kann und insgesamt die Menge M eines Dehydrierkatalysators umfasst, dass zum Betriebszeitpunkt t = to beim Durchgang des Rektionsgasgemischstroms durch das in Strömungsrichtung erste Drittel der Menge M ein Anteil von A mol-% der molaren Ausgangsmenge KW, beim Durchgang des Reaktionsgasgemischstroms durch das in Strömungsrichtung zweite Drittel der Menge M ein Anteil von B mol-% der molaren Ausgangsmenge KW, und beim Durchgang des Reaktionsgasgemischstroms durch das in Strömungsrichtung letzte Drittel der Menge M ein Anteil von C mol-% der molaren Ausgangsmenge KW des zu dehydrierenden Kohlenwasserstoffs zu dehydriertem Kohlenwasserstoff mit der Maßgabe umgesetzt wird, dass A > B > C gilt und beim Durchgang des Reaktionsgasgemischstroms durch das Katalysatorgesamtbett insgesamt G = (A + B + C) mol-% der in diesem enthaltenen molaren Ausgangsmenge KW an zu dehydrierendem Kohlenwasserstoff zu dehydriertem Kohlenwasserstoff dehydriert werden, wobei dem Reaktionsgasgemischstrom zwischen seinem Eintritt in den Beginn des Katalysatorgesamtbetts und seinem Austritt aus dem Ende des Katalysatorgesamtbetts gegebenenfalls Mengenströme an molekularem Sauerstoff, molekularem Wasserstoff, Wasserdampf und/oder sonstigem Inertgas als Dehydrierhilfsgase zugeführt werden, und der mit zunehmender Betriebsdauer in einem Betriebszeitpunktintervall t₀ < t < t_{R} einhergehenden Deaktivierung des Katalysatorgesamtbetts, wobei t_{R} der Betriebszeitpunkt t ist, zu dem die Dehydrierung unterbrochen und das Katalysatorgesamtbett hinter dem Betriebszeitpunkt t = t₀ erstmals regeneriert wird, dadurch entgegenwirkt, dass man den Verlauf der Temperatur des Reaktionsgasgemischstroms innerhalb des Katalysatorgesamtbetts und/oder den Mengenstrom gegebenenfalls zugeführter Dehydrierhilfsgase variiert,
**dadurch gekennzeichnet,**
**dass** die Variation so durchgeführt wird, dass mit zunehmendem Betriebszeitpunkt t der Anteil A abnimmt, der Anteil B ein Maximum durchläuft und der Anteil C zunimmt, und derart, dass der Gesamtumsatz G im Rahmen G ± 5 mol-% konstant bleibt.

2. Verfahren nach Anspruch 1, **dadurch gekennzeichnet, dass** der zu dehydrierende Kohlenwasserstoff ein C₂- bis C₆-Alkan und der dehydrierte Kohlenwasserstoff ein C₂- bis C₆-Alken ist.

3. Verfahren nach Anspruch 1, **dadurch gekennzeichnet, dass** der zu dehydrierende Kohlenwasserstoff n-Propan und der dehydrierte Kohlenwasserstoff Propylen ist.

4. Verfahren nach einem der Ansprüche 1 bis 3, **dadurch gekennzeichnet, dass** der die molare Ausgangsmenge KW an zu dehydrierendem Kohlenwasserstoff enthaltende Reaktionsgasgemischstrom zusätzlich Wasserdampf enthält.

5. Verfahren nach einem der Ansprüche 1 bis 4, **dadurch gekennzeichnet, dass** der die molare Ausgangsmenge KW an zu dehydrierendem Kohlenwasserstoff enthaltende Reaktionsgasgemischstrom zusätzlich molekularen Sauerstoff enthält.

6. Verfahren nach einem der Ansprüche 1 bis 5, **dadurch gekennzeichnet, dass** der die molare Ausgangsmenge KW an zu dehydrierendem Kohlenwasserstoff enthaltende Reaktionsgasgemischstrom zusätzlich molekularen Wasserstoff enthält.

7. Verfahren nach einem der Ansprüche 1 bis 6, **dadurch gekennzeichnet, dass** G zum Betriebszeitpunkt tₒ 10 bis 60 mol-% beträgt.

8. Verfahren nach einem der Ansprüche 1 bis 6, **dadurch gekennzeichnet, dass** G zum Betriebszeitpunkt tₒ 15 bis 40 mol-% beträgt.

9. Verfahren nach einem der Ansprüche 1 bis 6, **dadurch gekennzeichnet, dass** G zum Betriebszeitpunkt tₒ 15 bis 30 mol-% beträgt.

10. Verfahren nach einem der Ansprüche 1 bis 9, **dadurch gekennzeichnet, dass** zum Betriebszeitpunkt t = t₀, bezogen auf den Gesamtumsatz G = (A + B + C), der Teilumsatz A 45 bis 80 %, der Teilumsatz B 20 bis 40 % und der Teilumsatz C 0 bis 15 % beträgt.

11. Verfahren nach einem der Ansprüche 1 bis 9, **dadurch gekennzeichnet, dass** zum Betriebszeitpunkt t = tₒ, bezogen auf den Gesamtumsatz G = (A + B + C), der Teilumsatz A 55 bis 70 %, der Teilumsatz B 20 bis 35 % und der Teilumsatz C 7 bis 13 % beträgt.

12. Verfahren nach einem der Ansprüche 1 bis 11, **dadurch gekennzeichnet, dass** mit zunehmender Betriebsdauer innerhalb des Betriebszeitpunktintervalls tₒ < t < t_{R} der Teilumsatz A unter den Teilumsatz C fällt, so dass dann C > A gilt.

13. Verfahren nach einem der Ansprüche 1 bis 11, **dadurch gekennzeichnet, dass** mit zunehmender Betriebsdauer innerhalb des Betriebszeitpunktintervalls tₒ < t < t_{R} sich die Abfolge der Teilumsätze A, B, C von A > B > C ausgehend nach C > B > A umkehrt.

14. Verfahren nach einem der Ansprüche 1 bis 13, **dadurch gekennzeichnet, dass** der Wert A innerhalb des Betriebszeitpunktintervalls tₒ < t < t_{R} 20 % seines Wertes zum Betriebszeitpunkt t = tₒ nicht unterschreitet.

15. Verfahren nach einem der Ansprüche 1 bis 14, **dadurch gekennzeichnet, dass** die Werte B, C innerhalb des Betriebszeitpunktintervalls tₒ < t < t_{R} 95 % des Wertes von A zum Betriebszeitpunkt t = tₒ nicht überschreiten.

16. Verfahren nach einem der Ansprüche 1 bis 15, **dadurch gekennzeichnet, dass** die Reaktionszone RZ als adiabater Hordenreaktor mit gleichzeitiger Zuführung von Mengenströmen an einem molekularen Sauerstoff enthaltenden Dehydrierhilfsgas zwischen Horden ausgeführt ist.

17. Verfahren nach einem der Ansprüche 1 bis 15, **dadurch gekennzeichnet, dass** die Reaktionszone RZ als Hintereinanderschaltung von wenigstens zwei adiabat ausgeführten Dehydrierreaktoren mit gleichzeitiger Zuführung von Mengenströmen an einem molekularen Sauerstoff enthaltenden Dehydrierhilfsgas zwischen in der Hintereinanderschaltung unmittelbar aufeinanderfolgenden adiabat ausgeführten Dehydrierreaktoren ausgeführt ist.

18. Verfahren nach einem der Ansprüche 1 bis 17, **dadurch gekennzeichnet, dass** das Katalysatorgesamtbett ein Festbett ist.

19. Verfahren nach einem der Ansprüche 1 bis 18, **dadurch gekennzeichnet, dass** der Dehydrierkatalysator ein solcher ist, der wenigstens ein auf einem oxidischen Träger abgeschiedenes Metall aufweist.

20. Verfahren nach Anspruch 19, **dadurch gekennzeichnet, dass** das wenigstens eine Metall ein Element aus der Platingruppe ist.

21. Verfahren nach Anspruch 19, **dadurch gekennzeichnet, dass** das wenigstens eine Metall Platin ist.

22. Verfahren nach einem der Ansprüche 1 bis 21, **dadurch gekennzeichnet, dass** man aus der Reaktionszone RZ einen in der Reaktionszone RZ gebildeten dehydrierten Kohlenwasserstoff und in der Reaktionszone RZ nicht umgesetzten zu dehydrierenden Kohlenwasserstoff enthaltenden Produktgasstrom entnimmt und diesen Produktgasstrom als solchen oder nach Abtrennung wenigstens einer Teilmenge seiner vom dehydrierten Kohlenwasserstoff und vom zu dehydrierenden Kohlenwasserstoff verschiedenen Bestandteile zur Beschickung wenigsten eines Oxidationsreaktors verwendet und in diesem den darin enthaltenden dehydrierten Kohlenwasserstoff einer selektiven heterogen katalysierten partiellen Gasphasenoxidation mit molekularem Sauerstoff zu einem das Partialoxidationsprodukt enthaltenden Partialoxidationsproduktgasgemisch unterwirft.

23. Verfahren nach Anspruch 22, **dadurch gekennzeichnet, dass** der zu dehydrierende Kohlenwasserstoff Propan, der dehydrierte Kohlenwasserstoff Propylen und das Partialoxidationsprodukt Acrolein, Acrylsäure oder deren Gemisch ist.

24. Verfahren nach Anspruch 22, **dadurch gekennzeichnet, dass** man in einer Trennzone der selektiven heterogen katalysierten partiellen Gasphasenoxidation nachfolgend aus dem Partialoxidationsproduktgasgemisch Partialoxidationsprodukt abtrennt und von dem dabei verbleibenden, nicht umgesetzten zu dehydrierenden Kohlenwasserstoff, molekularen Sauerstoff sowie gegebenenfalls nicht umgesetzten dehydrierten Kohlenwasserstoff enthaltenden Restgas, wenigstens eine nicht umgesetzten zu dehydrierenden Kohlenwasserstoff enthaltende Teilmenge als Partialoxidationskreisgas in die Reaktionszone RZ rückführt.

25. Verfahren nach Anspruch 24, **dadurch gekennzeichnet, dass** man das Partialoxidationsprodukt in der Trennzone vom Partialoxidationsproduktgasgemisch durch Überführen in die kondensierte Phase abtrennt.

26. Verfahren nach Anspruch 25, **dadurch gekennzeichnet, dass** das Partialoxidationsprodukt Acrylsäure ist und die Überführung in die kondensierte Phase durch absorptive und/oder kondensative Maßnahmen erfolgt.

27. Verfahren nach Anspruch 26, **dadurch gekennzeichnet, dass** unter Anwendung wenigstens eines thermischen Trennverfahrens eine Abtrennung der Acrylsäure aus der kondensierten Phase vorgenommen wird.

28. Verfahren nach Anspruch 27, **dadurch gekennzeichnet, dass** das wenigstens eine thermische Trennverfahren eine kristallisative Abtrennung von Acrylsäure aus flüssiger Phase umfasst.

29. Verfahren nach Anspruch 28, **dadurch gekennzeichnet, dass** die kristallisative Abtrennung eine Suspensionskristallisation ist.

30. Verfahren nach Anspruch 27, **dadurch gekennzeichnet, dass** sich an die Abtrennung der Acrylsäure ein Verfahren der radikalischen Polymerisation anschließt, in welchem abgetrennte Acrylsäure zur Herstellung von Polymerisaten radikalisch einpolymerisiert wird.

31. Verfahren nach Anspruch 27, **dadurch gekennzeichnet, dass** sich an die Abtrennung der Acrylsäure ein Verfahren zur Herstellung von Acrylsäureestern anschließt, in welchem abgetrennte Acrylsäure mit einem Alkohol verestert wird.

32. Verfahren nach Anspruch 31, **dadurch gekennzeichnet, dass** sich an das Verfahren zur Herstellung eines Acrylsäureesters ein Verfahren der radikalischen Polymerisation anschließt, in welchem so hergestellter Acrylsäureester einpolymerisiert wird.

## Claims

1. A process for the long-term operation of a continuous heterogeneously catalyzed partial dehydrogenation of a hydrocarbon to be dehydrogenated to a dehydrogenated hydrocarbon, in which, for the purpose of the heterogeneously catalyzed partial dehydrogenation of the hydrocarbon to be dehydrogenated, a reaction gas mixture stream comprising it in a molar starting amount KW is conducted at elevated temperature through an overall catalyst bed which is disposed in a reaction zone RZ and may consist of a plurality of partial catalyst beds arranged in succession in flow direction of the reaction gas mixture stream and comprises in total the amount M of a dehydrogenation catalyst, in such a way that, at the operating time t = to, a proportion of A mol% of the molar starting amount KW is converted as the reaction gas mixture stream passes through the first third of the amount M in flow direction, a proportion of B mol% of the molar starting amount KW is converted as the reaction gas mixture stream passes through the second third of the amount M in flow direction, and a proportion of C mol% of the molar starting amount KW of the hydrocarbon to be dehydrogenated is converted to dehydrogenated hydrocarbon as the reaction gas mixture stream passes through the last third of the amount M in flow direction, with the proviso that A > B > C, and a total of G = (A + B + C) mol% of the molar starting amount KW of hydrocarbon to be dehydrogenated present therein is dehydrogenated to dehydrogenated hydrocarbon as the reaction gas mixture stream passes through the overall catalyst bed, and streams of molecular oxygen, molecular hydrogen, steam and/or other inert gas are optionally supplied as dehydrogenation auxiliary gases to the reaction gas mixture stream between its entry into the start of the overall catalyst bed and its exit from the end of the overall catalyst bed, and the deactivation of the overall catalyst bed which accompanies increasing operating time in an operating time interval of to < t < t_{R}, where t_{R} is the operating time t at which the dehydrogenation is interrupted and the overall catalyst bed is regenerated for the first time after the operating time t = to, is counteracted by varying the profile of the temperature of the reaction gas mixture stream within the overall catalyst bed and/or the stream of any dehydrogenation auxiliary gases supplied,
which comprises
performing the variation in such a way that, with increasing operating time t, proportion A decreases, proportion B passes through a maximum and proportion C increases, and such that the overall conversion G remains constant within the range of G ± 5 mol%.

2. The process according to claim 1, wherein the hydrocarbon to be dehydrogenated is a C₂- to C₆-alkane and the dehydrogenated hydrocarbon is a C₂- to C₆-alkene.

3. The process according to claim 1, wherein the hydrocarbon to be dehydrogenated is n-propane and the dehydrogenated hydrocarbon is propylene.

4. The process according to any of claims 1 to 3, wherein the reaction gas mixture stream comprising the molar starting amount KW of hydrocarbon to be dehydrogenated additionally comprises steam.

5. The process according to any of claims 1 to 4, wherein the reaction gas mixture stream comprising the molar starting amount KW of hydrocarbon to be dehydrogenated additionally comprises molecular oxygen.

6. The process according to any of claims 1 to 5, wherein the reaction gas mixture stream comprising the molar starting amount KW of hydrocarbon to be dehydrogenated additionally comprises molecular hydrogen.

7. The process according to any of claims 1 to 6, wherein G at the operating time t₀ is from 10 to 60 mol%.

8. The process according to any of claims 1 to 6, wherein G at the operating time t₀ is from 15 to 40 mol%.

9. The process according to any of claims 1 to 6, wherein G at the operating time t₀ is from 15 to 30 mol%.

10. The process according to any of claims 1 to 9, wherein, at the operating time t = to, based on the overall conversion G = (A + B + C), partial conversion A is from 45 to 80%, partial conversion B is from 20 to 40% and partial conversion C is from 0 to 15%.

11. The process according to any of claims 1 to 9, wherein, at the operating time t = to, based on the overall conversion G = (A + B + C), partial conversion A is from 55 to 70%, partial conversion B is from 20 to 35% and partial conversion C is from 7 to 13%.

12. The process according to any of claims 1 to 11, wherein, with increasing operating time within the operating time interval of to < t < t_{R}, partial conversion A falls below partial conversion C, so that C > A.

13. The process according to any of claims 1 to 11, wherein, with increasing operating time within the operating time interval to < t < t_{R}, the sequence of partial conversions A, B, C reverses starting from A > B > C to become C > B > A.

14. The process according to any of claims 1 to 13, wherein the value A does not go below 20% of its value at the operating time t = to within the operating time interval of to < t < t_{R}.

15. The process according to any of claims 1 to 14, wherein the values B, C do not exceed 95% of the value of A at the operating time t = to within the operating time interval of to < t < t_{R}.

16. The process according to any of claims 1 to 15, wherein the reaction zone RZ is configured as an adiabatic staged reactor with simultaneous supply of streams of a dehydrogenation auxiliary gas comprising molecular oxygen between stages.

17. The process according to any of claims 1 to 15, wherein the reaction zone RZ is designed as a series connection of at least two adiabatic dehydrogenation reactors with simultaneous supply of streams of a dehydrogenation auxiliary gas comprising molecular oxygen between immediately successive adiabatic dehydrogenation reactors in series connection.

18. The process according to any of claims 1 to 17, wherein the overall catalyst bed is a fixed bed.

19. The process according to any of claims 1 to 18, wherein the dehydrogenation catalyst is one which has at least one metal deposited on an oxidic support.

20. The process according to claim 19, wherein the at least one metal is an element from the platinum group.

21. The process according to claim 19, wherein the at least one metal is platinum.

22. The process according to any of claims 1 to 21, wherein a product gas stream comprising dehydrogenated hydrocarbon formed in the reaction zone RZ and hydrocarbon to be dehydrogenated which has not been converted in the reaction zone RZ is withdrawn from the reaction zone RZ, and this product gas stream, as such or after removal of at least a portion of its constituents other than the dehydrogenated hydrocarbon and the hydrocarbon to be dehydrogenated, is used to charge at least one oxidation reactor and the dehydrogenated hydrocarbon present therein is subjected in the reactor to a selective heterogeneously catalyzed partial gas phase oxidation with molecular oxygen to give a partial oxidation product gas mixture comprising the partial oxidation product.

23. The process according to claim 22, wherein the hydrocarbon to be dehydrogenated is propane, the dehydrogenated hydrocarbon is propylene and the partial oxidation product is acrolein, acrylic acid or a mixture thereof.

24. The process according to claim 22, wherein partial oxidation product is subsequently removed from the partial oxidation product gas mixture in a separation zone of the selective heterogeneously catalyzed partial gas phase oxidation, and at least a portion comprising unconverted hydrocarbon to be dehydrogenated of the remaining residual gas comprising unconverted hydrocarbon to be dehydrogenated and molecular oxygen, with or without unconverted dehydrogenated hydrocarbon, is recycled as a partial oxidation cycle gas into the reaction zone RZ.

25. The process according to claim 24, wherein the partial oxidation product is removed from the partial oxidation product gas mixture in the separation zone by conversion to the condensed phase.

26. The process according to claim 25, wherein the partial oxidation product is acrylic acid and the conversion to the condensed phase is effected by absorptive and/or condensative measures.

27. The process according to claim 26, wherein a removal of the acrylic acid from the condensed phase is undertaken using at least one thermal separation process.

28. The process according to claim 27, wherein the at least one thermal separation process comprises a crystallizative removal of acrylic acid from the liquid phase.

29. The process according to claim 28, wherein the crystallizative removal is a suspension crystallization.

30. The process according to claim 27, wherein the removal of the acrylic acid is followed by a process for free-radical polymerization, in which removed acrylic acid is polymerized free-radically to prepare polymers.

31. The process according to claim 27, wherein the removal of the acrylic acid is followed by a process for preparing acrylic esters, in which removed acrylic acid is esterified with an alcohol.

32. The process according to claim 31, wherein the process for preparing an acrylic ester is followed by a process for free-radical polymerization, in which acrylic ester thus prepared is polymerized.

## Revendications

1. Procédé de fonctionnement à long terme d'une déshydrogénation partielle sous catalyse hétérogène fonctionnant en continu d'un hydrocarbure à déshydrogéner en un hydrocarbure déshydrogéné, selon lequel, dans le but de la déshydrogénation partielle sous catalyse hétérogène de l'hydrocarbure à déshydrogéner, un courant de mélange gazeux réactionnel contenant celui-ci en une quantité initiale molaire KW est conduit à température élevée au travers d'un lit catalytique total se trouvant dans une zone de réaction RZ, qui peut être constitué par plusieurs lits catalytiques partiels agencés successivement dans la direction de l'écoulement du courant de mélange gazeux réactionnel et comprend au total la quantité M d'un catalyseur de déshydrogénation, de manière à ce qu'au point de fonctionnement t = t₀, lors du passage du courant de mélange gazeux réactionnel au travers du premier tiers dans la direction de l'écoulement de la quantité M, une proportion de A % en moles de la quantité initiale molaire KW, lors du passage du courant de mélange gazeux réactionnel au travers du deuxième tiers dans la direction d'écoulement de la quantité M, une proportion de B % en moles de la quantité initiale molaire KW, et lors du passage du courant de mélange gazeux réactionnel au travers du dernier tiers dans la direction de l'écoulement de la quantité M, une proportion de C % en moles de la quantité initiale molaire KW de l'hydrocarbure à déshydrogéner en hydrocarbure déshydrogéné aient été transformées, à condition que A > B > C, et lors du passage du courant de mélange gazeux réactionnel au travers du lit catalytique total, au total G = (A + B + C) % en moles de la quantité initiale molaire KW contenue dans celui-ci de l'hydrocarbure à déshydrogéner ait été déshydrogénée en hydrocarbure déshydrogéné, des débits massiques d'oxygène molaire, d'hydrogène moléculaire, de vapeur d'eau et/ou d'un autre gaz inerte étant éventuellement ajoutés en tant que gaz auxiliaires de déshydrogénation au courant de mélange gazeux réactionnel entre son entrée au début du lit catalytique total et sa sortie à la fin du lit catalytique total, et la désactivation du lit catalytique total qui accompagne la durée de fonctionnement croissante dans un intervalle de points de fonctionnement t₀ < t < t_{R}, t_{R} étant le point de fonctionnement t auquel la déshydrogénation est interrompue et le lit catalytique total est régénéré pour la première fois après le point de fonctionnement t = t₀, étant contrée par variation de l'évolution de la température du courant de mélange gazeux réactionnel dans le lit catalytique total et/ou du débit massique des gaz auxiliaires de déshydrogénation éventuellement ajoutés,
**caractérisé en ce que**
la variation est réalisée de manière à ce que la proportion A diminue, la proportion B passe par un maximum et la proportion C augmente avec la durée de fonctionnement t croissante et de manière à ce que la conversion totale G reste constante dans les limites de G ± 5 % en moles.

2. Procédé selon la revendication 1, **caractérisé en ce que** l'hydrocarbure à déshydrogéner est un alcane en C₂ à C₆ et l'hydrocarbure déshydrogéné est un alcène en C₂ à C₆.

3. Procédé selon la revendication 1, **caractérisé en ce que** l'hydrocarbure à déshydrogéner est le n-propane et l'hydrocarbure déshydrogéné est le propylène.

4. Procédé selon l'une quelconque des revendications 1 à 3, **caractérisé en ce que** le courant de mélange gazeux réactionnel contenant la quantité initiale molaire KW d'hydrocarbure à déshydrogéner contient également de la vapeur d'eau.

5. Procédé selon l'une quelconque des revendications 1 à 4, **caractérisé en ce que** le courant de mélange gazeux réactionnel contenant la quantité initiale molaire KW d'hydrocarbure à déshydrogéner contient également de l'oxygène moléculaire.

6. Procédé selon l'une quelconque des revendications 1 à 5, **caractérisé en ce que** le courant de mélange gazeux réactionnel contenant la quantité initiale molaire KW d'hydrocarbure à déshydrogéner contient également de l'hydrogène moléculaire.

7. Procédé selon l'une quelconque des revendications 1 à 6, **caractérisé en ce que** G au point de fonctionnement t₀ est de 10 à 60 % en moles.

8. Procédé selon l'une quelconque des revendications 1 à 6, **caractérisé en ce que** G au point de fonctionnement t₀ est de 15 à 40 % en moles.

9. Procédé selon l'une quelconque des revendications 1 à 6, **caractérisé en ce que** G au point de fonctionnement t₀ est de 15 à 30 % en moles.

10. Procédé selon l'une quelconque des revendications 1 à 9, **caractérisé en ce qu'**au point de fonctionnement t=t₀, par rapport à la conversion totale G = (A + B + C), la conversion partielle A est de 45 à 80 %, la conversion partielle B est de 20 à 40 % et la conversion partielle C est de 0 à 15 %.

11. Procédé selon l'une quelconque des revendications 1 à 9, **caractérisé en ce qu'**au point de fonctionnement t=t₀, par rapport à la conversion totale G = (A + B + C), la conversion partielle A est de 55 à 70 %, la conversion partielle B est de 20 à 35 % et la conversion partielle C est de 7 à 13 %.

12. Procédé selon l'une quelconque des revendications 1 à 11, **caractérisé en ce que** lorsque la durée de fonctionnement augmente dans l'intervalle de points de fonctionnement t₀ < t < t_{R}, la conversion partielle A chute sous la conversion partielle C, de manière à ce que C > A.

13. Procédé selon l'une quelconque des revendications 1 à 11, **caractérisé en ce que** lorsque la durée de fonctionnement augmente dans l'intervalle de points de fonctionnement t₀ < t < t_{R}, l'ordre des conversions partielles A, B, C s'inverse de A > B > C à C > B > A.

14. Procédé selon l'une quelconque des revendications 1 à 13, **caractérisé en ce que** la valeur A ne passe pas en dessous de 20 % de sa valeur au point de fonctionnement t = t₀ dans l'intervalle de points de fonctionnement t₀ < t < t_{R}.

15. Procédé selon l'une quelconque des revendications 1 à 14, **caractérisé en ce que** les valeurs B, C ne dépassent pas 95 % de la valeur de A au point de fonctionnement t = t₀ dans l'intervalle de points de fonctionnement t₀ < t < t_{R}.

16. Procédé selon l'une quelconque des revendications 1 à 15, **caractérisé en ce que** la zone de réaction RZ est configurée sous la forme d'un réacteur adiabatique à plateaux avec introduction simultanée de débits massiques d'un gaz auxiliaire de déshydrogénation contenant de l'oxygène moléculaire entre les plateaux.

17. Procédé selon l'une quelconque des revendications 1 à 15, **caractérisé en ce que** la zone de réaction RZ est configurée sous la forme d'une connexion en série d'au moins deux réacteurs de déshydrogénation adiabatiques avec introduction simultanée de débits massiques d'un gaz auxiliaire de déshydrogénation contenant de l'oxygène moléculaire entre des réacteurs de déshydrogénation adiabatiques directement successifs dans la connexion en série.

18. Procédé selon l'une quelconque des revendications 1 à 17, **caractérisé en ce que** le lit catalytique total est un lit fixe.

19. Procédé selon l'une quelconque des revendications 1 à 18, **caractérisé en ce que** le catalyseur de déshydrogénation est un catalyseur de déshydrogénation comprenant au moins un métal déposé sur un support oxydique.

20. Procédé selon la revendication 19, **caractérisé en ce que** le ou les métaux sont un élément du groupe du platine.

21. Procédé selon la revendication 19, **caractérisé en ce que** le ou les métaux sont le platine.

22. Procédé selon l'une quelconque des revendications 1 à 21, **caractérisé en ce qu'**un courant gazeux de produits contenant l'hydrocarbure déshydrogéné formé dans la zone de réaction RZ et l'hydrocarbure à déshydrogéner non réagi dans la zone de réaction RZ est soutiré de la zone de réaction RZ et ce courant gazeux de produits est utilisé en tant que tel ou après la séparation d'au moins une partie de ses constituants différents de l'hydrocarbure déshydrogéné et de l'hydrocarbure à déshydrogéner pour l'alimentation d'au moins un réacteur d'oxydation, et l'hydrocarbure déshydrogéné qu'il contient est soumis dans celui-ci à une oxydation partielle sélective sous catalyse hétérogène en phase gazeuse avec de l'oxygène moléculaire pour former un mélange gazeux de produits de l'oxydation partielle contenant le produit de l'oxydation partielle.

23. Procédé selon la revendication 22, **caractérisé en ce que** l'hydrocarbure à déshydrogéner est le propane, l'hydrocarbure déshydrogéné est le propylène et le produit de l'oxydation partielle est l'acroléine, l'acide acrylique ou leur mélange.

24. Procédé selon la revendication 22, **caractérisé en ce que** le produit de l'oxydation partielle est séparé du mélange gazeux de produits de l'oxydation partielle dans une zone de séparation suivant l'oxydation partielle sélective sous catalyse hétérogène en phase gazeuse et, à partir du gaz résiduel restant contenant l'hydrocarbure à déshydrogéner non réagi, l'oxygène moléculaire et éventuellement l'hydrocarbure déshydrogéné non réagi, au moins une partie contenant l'hydrocarbure à déshydrogéner non réagi est recyclée en tant que gaz de recyclage de l'oxydation partielle dans la zone de réaction RZ.

25. Procédé selon la revendication 24, **caractérisé en ce que** le produit de l'oxydation partielle est séparé du mélange gazeux de produits de l'oxydation partielle dans la zone de séparation par transfert dans la phase condensée.

26. Procédé selon la revendication 25, **caractérisé en ce que** le produit de l'oxydation partielle est l'acide acrylique et le transfert dans la phase condensée a lieu par des mesures d'absorption et/ou de condensation.

27. Procédé selon la revendication 26, **caractérisé en ce qu'**une séparation de l'acide acrylique de la phase condensée est réalisée en utilisant au moins un procédé de séparation thermique.

28. Procédé selon la revendication 27, **caractérisé en ce que** le ou les procédés de séparation thermiques sont une séparation par cristallisation de l'acide acrylique de la phase liquide.

29. Procédé selon la revendication 28, **caractérisé en ce que** la séparation par cristallisation est une cristallisation en suspension.

30. Procédé selon la revendication 27, **caractérisé en ce que** la séparation de l'acide acrylique est suivie par un procédé de polymérisation radicalaire lors duquel l'acide acrylique séparé est polymérisé par voie radicalaire pour la fabrication de polymères.

31. Procédé selon la revendication 27, **caractérisé en ce que** la séparation de l'acide acrylique est suivie par un procédé de fabrication d'esters de l'acide acrylique lors duquel l'acide acrylique séparé est estérifié avec un alcool.

32. Procédé selon la revendication 31, **caractérisé en ce que** le procédé de fabrication d'un ester de l'acide acrylique est suivi par un procédé de polymérisation radicalaire lors duquel l'ester de l'acide acrylique ainsi fabriqué est polymérisé.
